# EUROPEAN PATENT SPECIFICATION

(11) **EP 3 436 595 B1**
(45) Date of publication and mention of the grant of the patent: **28.02.2024**
(21) Application number: 17714445.8
(22) Date of filing: 30.03.2017
(51) Int. Cl.: C12P 13/22, C12P 17/10

(54) **OPTIMIZED MICROBIAL CELLS FOR PRODUCTION OF MELATONIN AND OTHER COMPOUNDS**
OPTIMIERTE MIKROBIELLE ZELLEN ZUR HERSTELLUNG VON MELATONIN UND ANDEREN VERBINDUNGEN
CELLULES MICROBIENNES OPTIMISÉES POUR LA PRODUCTION DE MÉLATONINE ET D'AUTRES COMPOSÉS

(30) Priority: 31.03.2016 US 201662315864 P; 19.05.2016 EP 16170405
(43) Date of publication of application: 06.02.2019
(73) Proprietor: Danmarks Tekniske Universitet, 2800 Kongens Lyngby (DK)
(72) Inventor: LUO, Hao, 2860 Søborg (DK); FÖRSTER, Jochen, 1700 Copenhagen V (DK)
(74) Representative: Inspicos P/S
(86) International application number: PCT/EP2017/057520
(87) International publication number: WO 2017/167866

(56) References cited:
- WO-A1-2013/127914
- WO-A1-2015/032911
- US-A1- 2012 246 748
- DATABASE Protein [Online] 1 October 2015 (2015-10-01), "GTP cyclohydrolase I [Shigella sonnei]", XP002770265, retrieved from NCBI Database accession no. WP_053008051.1
- DATABASE Protein [Online] 27 February 2016 (2016-02-27), "GTP cyclohydrolase I FolE [Escherichia coli]", XP002770266, retrieved from NCBI Database accession no. WP_061336373.1
- DATABASE Protein [Online] 1 October 2015 (2015-10-01), "GTP cyclohydrolase I [Escherichia coli]", XP002770267, retrieved from NCBI Database accession no. WP_032358670.1
- DATABASE Protein [Online] 22 March 2016 (2016-03-22), "GTP cyclohydrolase I FolE [Escherichia coli]", XP002770268, retrieved from NCBI Database accession no. WP_001649026
- DATABASE Protein [Online] 15 January 2016 (2016-01-15), "type I GTP cyclohydrolase I FolE [Erwinia teleogrylli]", XP002770269, retrieved from NCBI Database accession no. WP_058913807.1
- DATABASE Protein [Online] 22 March 2016 (2016-03-22), "GTP cyclohydrolase I FolE (Escherichia coli)", XP002760019, retrieved from NCBI Database accession no. WP_001639660
- DATABASE UniProt [Online] 9 May 2003 (2003-05-09), "RecName: Full=Tryptophan 5-hydroxylase 2; EC=1.14.16.4; AltName: Full=Neuronal tryptophan hydroxylase; AltName: Full=Tryptophan 5-monooxygenase 2;", XP002770270, retrieved from EBI accession no. UNIPROT:Q8IWU9 Database accession no. Q8IWU9
- DATABASE Protein [Online] 18 February 2014 (2014-02-18), "PREDICTED: tryptophan 5-hydroxylase 2 isoform X1 [Elephantulus edwardii]", XP002770271, retrieved from NCBI Database accession no. XP_006882629
- DATABASE UniProt [Online] 1 October 2002 (2002-10-01), "SubName: Full=Tryptophane hydroxylase {ECO:0000313|EMBL:AAK59708.1};", XP002760020, retrieved from EBI accession no. UNIPROT:Q8K3R1 Database accession no. Q8K3R1
- Susanne M Germann ET AL: "Glucose-based microbial production of the hormone melatoninin yeast Saccharomyces cerevisiae", Biotechnol. J, vol. 11 25 January 2016 (2016-01-25), pages 717-724, XP055373696, DOI: 10.1002/biot.201500143 Retrieved from the Internet: URL:https://www.ncbi.nlm.nih.gov/pmc/artic les/PMC5066760/pdf/BIOT-11-717.pdf [retrieved on 2017-05-17]

## Description

### FIELD OF THE INVENTION

The present invention relates to variants of *E. coli* GTP cyclohydrolase I (GCH1) and variants of a tryptohan hydroxylase (TPH), nucleic acid sequences encoding such variants, recombinant microbal cells comprising such variants or nucleic acid sequences, as well as methods for producing oxidation products of aromatic amino acids, such as, e.g., 5HTP, comprising culturing such recombinant cells. More specifically, the present invention relates to microbial host cells repurposed for optimal monooxygenase, *e.g*., amino acid hydroxylase, function, and to methods of using such cells.

### BACKGROUND OF THE INVENTION

Melatonin is a hormone secreted by the pineal gland in the brain which, inter alia, maintains the body's circadian rhythm, is involved regulating other hormones, and is a powerful antioxidant. Because of, *e.g*., its role in regulating circadian rhythm, melatonin has been available for many years as an over-the-counter dietary supplement in the U.S. This melatonin is, however, typically chemically synthesized, and there is a need for a simplified and more cost-effective procedure.

In animals, melatonin is biosynthesized from the native metabolite L-tryptophan via the intermediates 5-hydroxy-L-tryptophan (5HTP), serotonin and N-acetylserotonin. The first step is this pathway, the conversion of L-tryptophan to 5HTP, is catalyzed by L-tryptophan hydroxylase (TPH). TPH and many other mammalian aromatic amino acid hydroxylases require oxygen and tetrahydropterin (BH4) as cofactors.

WO 2013/127914 A1, WO 2013/127915 A1 and WO 2015/032911 A1 (Danmarks Tekniske Universitet) describe the production of 5HTP or melatonin in recombinant *E. coli* and S. *cerevisiae* cells expressing a TPH and enzymes of a biosynthetic pathway for producing the BH4 co-factor. BH4 does not occur naturally in *E. coli* or S. *cerevisiae* but can be biosynthesized from endogenous GTP via a pathway comprising a GTP cyclohydrolase I (GCH1), a 6-pyruvoyl-tetrahydropterin synthase (PTS or PTPS) and a sepiapterin reductase (SRP) (Yamamoto *et al.,* 2003; Ehrenworth *et al.,* 2015), and regenerated into BH4 via consecutive reactions by pterin-4a-carbolamine dehydratase (PCD) and dihydropterin reductase (DHPR) (*e.g*., WO 2015/032911 and Ehrenworth *et al.,* 2015). Nar *et al.* (1995) and Rebelo *et al.* (2003) have reported on the structure of the *E. coli* GCH1; FolE. The amino acid sequence of FolE is reported in UniProtKB P0A6T5 (SEQ ID NO: 16). An alternative FolE sequence is provided by NCBI Reference Sequence WP_001639660.

US 2014/134689 AA (University of California) describes methods of producing oxidation products such as L-DOPA, 5HTP, serotonin and/or melatonin from aromatic amino acids in a host cell which can, *e.g*., be capable of biosynthesizing BH4 or tetrahydromonapterin (MH4) from GTP. MH4 was reportedly capable of replacing THB as cofactor for tyrosine hydroxylase (TH).

MH4 is endogenous to, *e.g., E. coli* and *P. aeruginosa* cells, where it is hypothesized to be the cofactor for phenylalanine hydroxylase (PheH), its formation requiring FolX and FolM (Pribat *et al.,* 2010a). Phenylalanine hydroxylases from non-flowering plants, however, uniquely preferred 10-formyltetrahydrofolate (10-THF) as cofactor over BH4 and were not capable of using MH4 (Pribat *et al.,* 2010b).

Lin *et al.* (2014) describes that *E. coli* transformed with engineered bacterial phenylalanine 4-hydroxylases and a "regeneration" pathway based on a PCD and endogenous folM (dihydromonapterin reductase; DHMR) could convert tryptophan to 5HTP using endogenous MH4 as a co-factor. Hara and Kino (2013) produced 5HTP in a similar *E. coli* system, reportedly increasing the yield with the addition of BH4 cofactor.

Despite these and other advances in the art, there is still a need for recombinant microorganisms capable of efficient production of melatonin and related compounds. It is an object of the invention to provide such microorganisms.

### SUMMARY OF THE INVENTION

It has been found by the present inventor that, surprisingly, oxidation products of amino acids, such as 5HTP, can be produced in a recombinant microbial cell comprising a heterologous monooxygenase, such as a TPH, and a heterologous PCD. Optionally, the microbial cell further comprises other enzymes for converting the oxidation product into a desired end-product. For example, the recombinant microbial cell may comprise heterologous enzymes converting 5HTP into serotonin and/or melatonin. In some embodiments, this is possible even in the absence of exogenous nucleic acid encoding a DHPR or a DHMR. While not being limited to theory, this suggests that native *E. coli* compounds may support the monooxygenase (TPH) activity.

The present inventor has also identified variants of GCH1 which improve monooxygenase activity in recombinant microbial cells comprising a monooxygenase and a PCD, as well as variants of TPH with improved hydroxylation activity.

So, the present invention relates to variants of *E. coli* GTP cyclohydrolase I (GCH1) and tryptohan hydroxylase (TPH) enzymes, as well as to nucleic acids and vectors encoding such variants, to recombinant microbial cells expressing such variants, and to the use of such enzymes and recombinant microbial cells for producing oxidation products.

The variant of *E. coli* GTP cyclohydrolase I (GCH1) comprises one or more mutations, wherein,
in an *E. coli* cell comprising a pterin-4a-carbinolamine dehydratase (PCD) and at least one of a tryptophan hydroxylase (TPH), a tyrosine hydroxylase (TH) and a phenylalanine hydroxylase (PheH), the variant provides for an increased hydroxylation activity of at least one of the TPH, TH and PheH as compared to native *E. coli* GCH1,
wherein
   (i) the variant has at least 94% sequence identity to native *E. coli* GCH1 having the sequence of SEQ ID NO: 16 and at least one of the one or more mutations is in an amino acid residue selected from the group consisting of D97, M99, T101, V102, A125, K129, N170, V179, T196, S199, L200, S207, H212, E213, F214, L215 and H221,
      wherein the mutation in N170 is N170K, N170D or N170L; the mutation in V179 is V179A; the mutation in H212 is H212R or H212K; and the mutation in H221 is H221R or H221K; or
   (ii) the variant has at least 98% sequence identity to native *E. coli* GCH1 having the sequence of SEQ ID NO: 16 and at least one of the one or more mutations is in the amino acid residue T198, wherein the mutation in T198 is not T198P.

The variant of a tryptohan hydroxylase (TPH) comprises
a segment corresponding to residues E147 to T460 of *Homo sapiens* TPH having the sequence or SEQ ID NO:3,
an N-terminal methionine residue, and
a mutation in each residue corresponding to residues E147, N242 and P244 in SEQ ID NO:3,
wherein the variant is a variant of a *Homo sapiens* (SEQ ID NO:3), *Schistosoma mansoni* (SEQ ID NO:9), *Gallus gallus* (SEQ ID NO:6), *Sus scrofa* (SEQ ID NO:5), *Equus caballus* (SEQ ID NO:8), *Bos taurus* (SEQ ID NO:4), *Mus musculus* (SEQ ID NO:7) or *Oryctolagus cuniculus* (SEQ ID NO:1):TPH, and wherein the segment in the variant has at least about 80% sequence identity to the segment in the native sequence,
wherein the variant provides for a higher tryptophan hydroxylation activity than the native TPH, and
wherein
   (a) the mutation in the residue corresponding to residue E147 is an amino acid substitution selected from 147K, 147R and 147H;
   (b) the mutation in the residue corresponding to residue N242 is the amino acid substitution 242I; and
   (c) the mutation in the residue corresponding to P244 is selected from 244C, 244D, 244L and 244Q.

To provide for production of other compounds of interests, the recombinant microbial cell can further comprise nucleic acids encoding other enzymes. For example, to produce compounds such as serotonin, N-acetyl-serotonin and melatonin, the recombinant microbial cell may comprise heterologous nucleic acid sequences encoding a 5HTP decarboxylase (ADDC), a serotonin acetyltransferase (AANAT) and/or an acetylserotonin O-methyltransferase (ASMT), as described further below.

In further aspects, the invention relates to methods of producing 5HTP, L-DOPA or tyrosine, as well as downstream products such as, *e.g.,* serotonin, melatonin and hydroxytyrosol, using such recombinant microbial cells.

These and other aspects and embodiments are described in more detail below.

### LEGENDS TO THE FIGURES

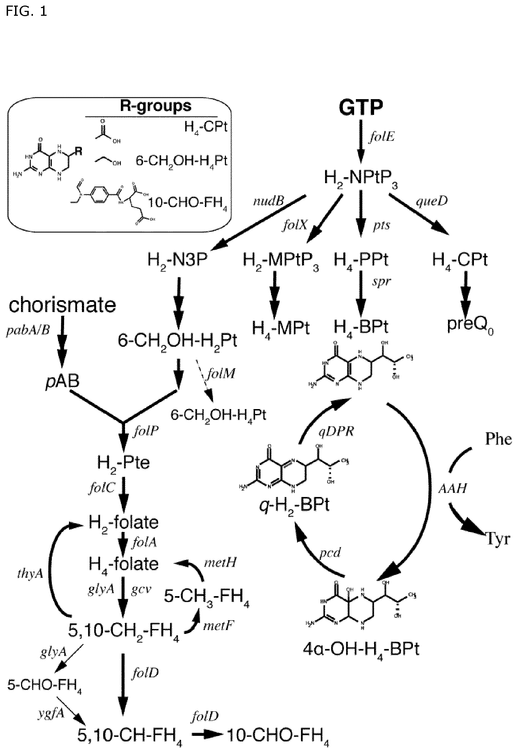

Fig. 1: An overview of pterin and folic acids biosynthesis from GTP in *E. coli.* Black solid arrows indicate native *E. coli* biosynthesis pathways of folic acids, tetrahydromonapterin (H₄-MPt), and preQ₀ from a common precursor, dihydroneopterin triphosphate (H₂-NPtP₃). The heterologous tetrahydrobiopterin (H₄-BPt) biosynthesis and recycling pathways are also shown. Arrow in dashed line shows a putative reaction. Thin arrows represent formation of 5-formyl-tetrahydrofolate (5-CHO-FH₄) in a side reaction of GlyA and recycling via Ygfa. The insert shows selected pterin and folate compounds in *E. coli* which share a common head group with H₄-BPt. Genes are labeled in italics. Other abbreviations: aromatic amino acid hydroxylase (AAH), dihydroneopterin-3-phosphate (H₂-N₃P), dihydromonapterin triphosphate (H₂-MPtP₃), 6-pyruvoyl-tetrahydrobiopterin (H₄-PPt), 6-carboxy-tetrahydropterin (H₄-CPt), quinonoid dihydrobiopterin (q-H₂-BPt), 4α-OH-H₄-BPt (pterin-4a-carbinolamine), dihydropteroate (H₂-Pte), dihydrofolate (H₂-folate), tetrahydrofolate (H₄-folate), 5-methyl-tetrahydrofolate (5-CH₃-FH₄), 5,10-methenyltetrahydrofolate (5,10-CH₂-FH₄), 5,10-methylenetetrahydrofolate (5,10-CH-FH₄), 10-formyl-tetrahydrofolate (10-CHO-FH₄), and *p-*aminobenzoate (*p*AB).

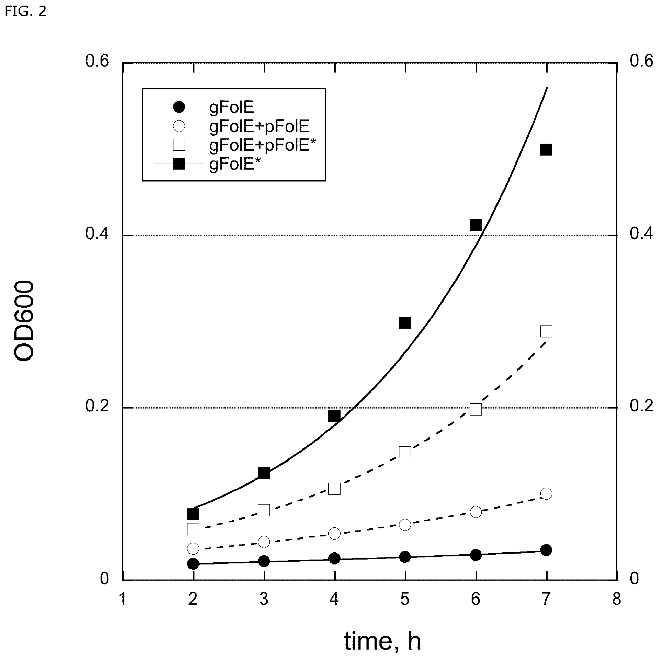

Fig. 2: FolE(T198I) mutation cannot be compensated by overexpression. TpH-dependent cell growth were evaluated under four conditions. 1) a single-copy native chromosomal FolE (gFolE), 2) a single-copy native chromosomal FolE and a native copy expressed from plasmid (gFolE+pFolE), 3) a single-copy native chromosomal FolE and a mutant copy expressed from plasmid (gFolE+pFolE*) and 4) a single-copy mutant chromosomal FolE (gFolE*)

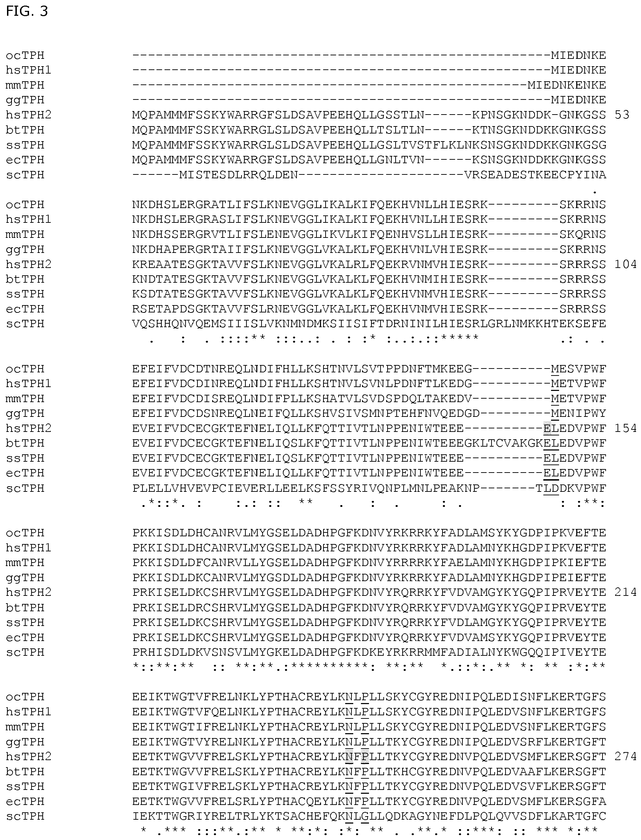

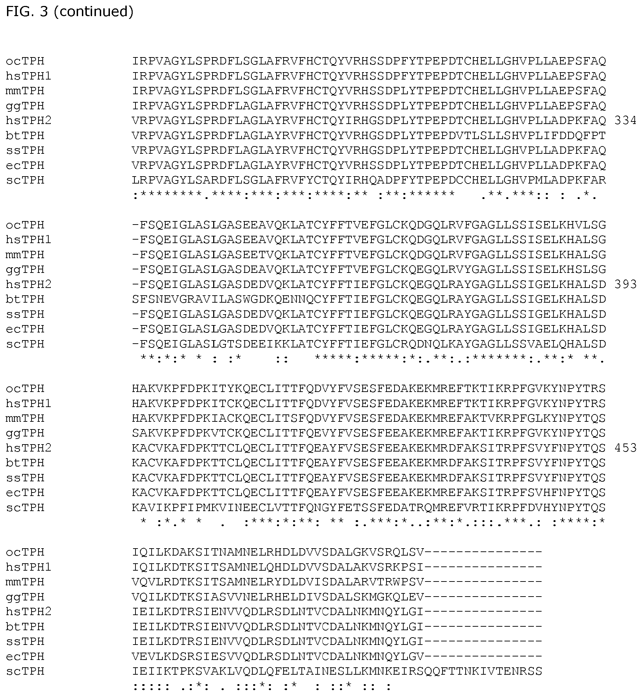

Fig. 3: ClustalW alignment of ocTPH (SEQ ID NO: 1), hsTPH1 (SEQ ID NO:2), mmTPH (SEQ ID NO:7), ggTPH (SEQ ID NO:6), hsTPH2 (SEQ ID NO:3), btTPH (SEQ ID NO:), ssTPH (SEQ ID NO:5), ecTPH (SEQ ID NO:8) and scTPH (SEQ ID NO:5) indicating, in underlined text, the residues corresponding to residues E147, L148, N242 and P244 in Homo sapiens TPH2 (SEQ ID NO:3).

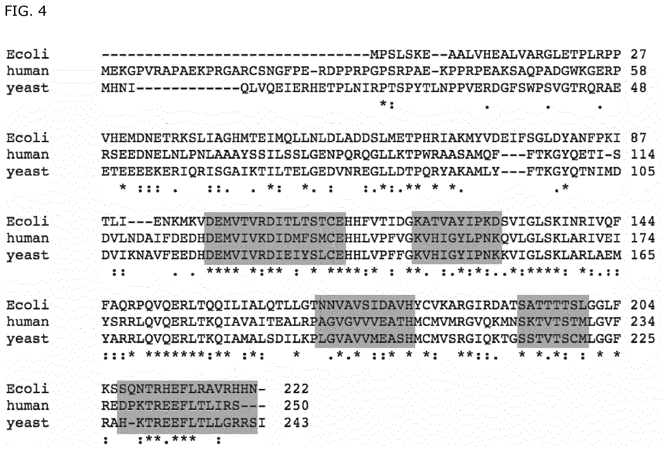

Fig. 4: Alignment of GCH1 from *E. coli* ("Ecoli," SEQ ID NO:16), *S*. *cerevisiae* ("yeast"; SEQ ID NO: 17) and *Homo sapiens* ("human", SEQ ID NO: 14), indicating the residues corresponding to residues D97-E112, K121-D130, N170-H180, S193-L200 and S207-N222 in *E. coli* GCH1 (SEQ ID NO: 16).

### DETAILED DISCLOSURE OF THE INVENTION

The present invention provides a recombinant microbial cell comprising nucleic acid sequences, optionally heterologous nucleic acid sequences, encoding a monooxygenase, a PCD and a GCH1. As shown by the results in Example 1, surprisingly, it is possible to use native bacterial THB-resembling species to support heterologous, optionally mammalian, TPH activity, with functional expression of a heterologous PCD gene being the only requirement. Additionally, by laboratory evolution, it was further possible to identify specific mutations in, e.g., GCH1 providing for a 10-fold increase in 5HTP production as compared to the parent *E*. *coli* cell.

In one aspect, the invention provides the variant of *E. coli* GTP cyclohydrolase I (FolE). Non-limiting examples according to this aspect are those wherein the variant
(a) comprises a mutation selected from D97V, D97L, D97A, D97T, M99C, M99T, M99V, M99L, M99I, T101I, T101V, T101L, V102M, N170K, N170D, N170L, V179A, V179M, T196I, T196V, T196L, S199Y, S199F, L200P, L200C, L200S, L200A, S207R, S207K, S207M, H212R, H212K, E213K, E213R, F214A, F214G, F214S, L215P, L215Q, L215N, L215D, L215T, L215S, L215G, L215A, L215C, L215F, L215M, H221R and H221K, or a combination thereof;
(b) has at least about 98% sequence identity to native *E. coli* GCH1;
(c) provides for a hydroxylation activity of at least about 120%, such as at leat 130%, as compared to native *E. coli* GCH1; or
(d) a combination of any two or more of (a) to (c).

Other non-limiting examples according to this aspect are those wherein the variant
(a) comprises a mutation selected from T198I, T198V, T198S and T198L;
(b) has at least 99% sequence identity to the native *E. coli* GCH1;
(c) provides for a hydroxylation activity of at least about 120% as compared to native *E. coli* GCH1; or
(d) a combination of any two or more of (a) to (c).

Preferably, the GCH1 variant of any such aspect or embodiment comprises a mutation selected from T198I, T198S, F214S, V179A, M99I, L200P and L215P. The mutation may, for example, be T198I.

In another aspect, the invention provides the variant of a TPH. The mutation in the residue corresponding to residue E147 is an amino acid substitution selected from 147K, 147R and 147H; the mutation in the residue corresponding to residue N242 is an amino acid substitution which is 242I; and the mutation in the residue corresponding to P244 is selected from 244C, 244D, 244L and 244Q, *e.g.,* 244C or 244D, such as 244D.

In another aspect, the invention provides a nucleic acid sequence encoding such variant *E*. *coli* GCH1, *Homo sapiens* TPH, or both, optionally in the form of one or more vectors which may further comprise one or more expression control sequences. The coding sequence of a variant *Homo sapiens* TPH may, for example, have the 5'-end *atgaaa* and be operably linked to a Trc promoter.

In another aspect, the invention provides a recombinant microbial cell comprising a variant *E*. *coli* GTP GCH1 as described herein or a nucleic acid sequence encoding such a variant. The recombinant microbial cell may further comprise nucleic acid sequences, optionally heterologous, encoding a monooxygenase and a PCD. In preferred embodiments, the recombinant microbial cell is one wherein
(a) the PCD is selected from *Chromobacterium violecum, Homo sapiens, Pseudomonas aeruginosa* and *Rattus norvegicus*; or a functionally active variant, homolog or fragment thereof;
(b) the monooxygenase is a TPH selected from a *Schistosoma mansoni, Homo sapiens, Gallus gallus, Bos taurus, Sus scrofa, Equus caballus, Mus musculus* and *Oryctolagus cuniculus* TPH; or a functionally active variant, homolog or fragment of any thereof;
*(c)* the monooxygenase is a TH selected from *Rattus norwegicus, Homo sapiens, Mus musculus, Bos taurus, Gallus gallus* or a functionally active variant, homolog or fragment thereof;
(d) the monooxygenase is a PheH selected from *Chromobacterium violaceum, Xanthomonas campestris pv. Viticola, Pseudomonas aeruginosa, Pseudomonas putida, Homo sapiens, Mus musculus, Streptomyces coeruleorubidus* or a functionally active variant, homolog or fragment thereof; or
(e) a combination of (a) and (b), (a) and (c) or (a) and (d).

In some embodiments, each nucleic acid sequence is operably linked to an inducible, a regulated or a constitutive promoter, and/or at least one nucleic acid encoding an enzyme (*e.g.,* the GCH1 variant), is chromosomally integrated.

In some embodiments, the recombinant microbial cell further comprises
(a) a nucleic acid sequence encoding a 5HTP decarboxylase (ADDC);
(b) nucleic acid sequences encoding an ADDC and a serotonin acetyltransferase (AANAT);
(c) nucleic acid sequences encoding an ADDC, an AANAT, and an acetylserotonin O-methyltransferase (ASMT); and/or
(d) nucleic acid sequences encoding a dopa decarboxylase, a tyramine oxidase and an alcohol dehydrogenase.

The invention also provides a method of producing one or more oxidation products of an aromatic amino acid, comprising culturing the recombinant microbial cell of any aspect or embodiment in a medium comprising a carbon source, and, optionally, isolating the oxidation product. The oxidation product may, for example, comprise at least one of 5HTP, L-DOPA, tyrosine, m-tyrosine, serotonin, melatonin and hydroxytyrosol.

### Definitions

Unless otherwise specified or contradicted by context, amino acid residue numbers in *Homo sapiens* TPH2 herein refer to their position in the sequence provided by NCBI accession No. NP_775489 and UniprotKB reference Q8IWU9 (SEQ ID NO:3).

Unless otherwise specified or contradicted by context, amino acid residue numbers in *E. coli* GCH1 (FolE) herein refer to their position in the sequence provided by UniprotKB reference POA6T5 (SEQ ID NO: 16).

As used herein, "exogenous" means that the referenced item, such as a molecule, activity or pathway, is added to or introduced into the host cell or microorganism. An exogenous nucleic acid sequence can, for example, be introduced either as chromosomal genetic material by integration into a host chromosome or as non-chromosomal genetic material such as a plasmid. Such an exogenous nucleic acid sequence can encode an enzyme or enzyme activity which is either heterologous to the host cell or organism in question or which is an endogenous enzyme or enzyme activity in the host cell or organism. Likewise, an exogenous molecule such as a substrate or cofactor can be added to or introduced into the host cell or microorganism, *e.g.,* via adding the molecule to the media in or on which the host cell or microorganism resides.

In the present context the term "heterologous" means that the referenced item, such as a molecule, activity or pathway, does not normally appear in the host cell or microorganism species in question. Typically, a heterologous pathway comprises at least one nucleic acid sequence, enzyme or other component which is heterologous to the host cell.

As used herein, the terms "native" or "endogenous" mean that the referenced item is normally present in or native to the host cell or microbial species in question.

As used herein, "upregulating" an endogenous gene means increasing the transcription and/or translation of a gene present in the native host cell genome relative to a control, such as *e.g.* the unmodified host cell. Methods of upregulating genes are known in the art and include, *e.g.,* introducing a non-native promoter increasing transcription, modifying the native promoter, deleting genes encoding repressor protein, introducing multiple copies of the gene of interest, etc. "Downregulating" an endogenous gene as used herein means to reduce, optionally eliminate, the transcription or translation of an endogenous gene relative to a control, such as, *e.g.,* the unmodified host cell. Methods of down-regulating, disrupting and deleting genes are known to those of skill in the art, and include, *e.g.,* site-directed mutagenesis, genomic modifications based on homologous recombination, RNA degradation based on CAS9, etc.

In the present context, "overexpressing" refers to introducing an exogenous nucleic acid sequence encoding an enzyme which is either heterologous or native to the microbial host cell, or is a functionally (i.e., catalytically) active fragment or variant thereof, and expressing the exogenous nucleic acid sequence to increase the enzyme activity in the microbial cell as compared to the microbial host cell without the introduced exogenous nucleic acid sequence, *e.g.,* a native microbial host cell. This can be useful if, *e.g.,* a microbial host cell does not normally contain the enzymatic activity referred to, where the native enzymatic activity is insufficient, or the native enzyme is subjected to unwanted regulation. In such cases, an exogenous nucleic acid sequence encoding an enzyme which is heterologous to the microbial host cell and which has the desired activity and regulation patterns can be introduced. Overexpression of a nucleic acid sequence can be achieved by placing the nucleic acid sequence under the control of a strong promoter. Non-limiting examples of strong promoters suitable for, *e.g., E. coli* cells are Ptrc, Plac, PlacUV5, PT7, and PTrp. Non-limiting examples of strong promoters suitable for, e.g., yeast cells are TEF1, PGK1, HXT7 and TDH3.

As used herein, a gene that is a "homolog" or "homologous" to another gene is generally an ortholog (*i.e*., a descended from the same ancestral sequence but separated when a species diverges into two separate species) or a paralog (*i.e*., separated by gene duplication within a genome). Typically, homologous genes encode proteins with a moderate to high sequence identity (e.g., at least about 30%, such as at least about 50%, such as at least about 60%, such as at least about 70%, such as at least about 80%, such as at least about 90%, such as at least about 95%, such as at least about 99%, over at least the catalytically active portion, optionally over the full length) and/or can at least partially substitute for the other protein in terms of function, when transferred from one species into another. Homologs of a particular gene can be identified using publicly available and specialized biological databases, e.g., by the eggNOG, InParanoid, OrthoDB, OrthoMCL, OMA, Roundup, TreeFam, LOFT, Ortholuge, EnsemblCompara GeneTrees and HomoloGene.

A "variant" of a protein comprises one or more mutations, such as amino acid substitutions, insertions and deletions as compared to the parent or reference enzyme. Typically, the variant has a high sequence identity to the parent or reference enzyme (*e.g.,* at least about 80%, such as at least about 90%, such as at least about 91%, 92%, 93%, 94%, 95%, 96%, 97% or 98%, such as at least about 99%, over at least a catalytically active portion, optionally over the full length of the mature form of the reference enzyme, excluding signal peptide sequences and the like). As used herein, a variant has less than 100% sequence identity over the full-length of the mature form of the parent or reference enzyme. A catalytically active portion of *Homo sapiens* TPH2 may, for example, correspond to residues E147 to T460 of *Homo sapiens* TPH2 (SEQ ID NO:3; NP_775489.2), wherein the term "corresponds to" a certain reference residue means that the residue aligns with the residue when using a standard dual or multiple sequence alignment program such as, *e.g.,* ClustalW (available at, *e.g.,* www.genome.jp) or ClustalOmega (available at, *e.g.,* www.ebi.ac.uk), typically using the default settings.

A "fragment" of a protein comprises at least the part of the protein which is responsible for its function of interest, *e.g.,* in the case of an enzyme, its catalytic part for the enzymatic activity of interest. Typically, a "fragment" comprises a segment corresponding to at least about 30%, such as at least about 50%, such as at least about 60%, such as at least about 70%, such as at least about 80%, such as at least about 90%, such as at least about 95%, of the full-length protein.

A "functionally active" or "catalytically active" variant or fragment comprises mutations or deletions, respectively, which do not substantially affect the function or catalytic activity of the variant or fragment as compared to the parent or reference protein and can substitute at least partially for the parent or reference protein in terms of the function of interest. Typically, unless used in the context of mutations in specific TPH or GHC1 (FolE) amino acid residues according to the invention, a variant or fragment has a function, as determined by a suitable activity assay, of 80-120%, such as 90%-110%, such as 95%-105%, of the parent or reference protein.

As used herein, "vector" refers to any genetic element capable of serving as a vehicle of genetic transfer, expression, or replication for a exogenous nucleic acid sequence in a host cell. For example, a vector may be an artificial chromosome or a plasmid, and may be capable of stable integration into a host cell genome, or it may exist as an independent genetic element (*e.g.,* episome, plasmid). A vector may exist as a single nucleic acid sequence or as two or more separate nucleic acid sequences. Vectors may be single copy vectors or multicopy vectors when present in a host cell. Preferred vectors for use in the present invention are expression vector molecules in which one or more functional genes can be inserted into the vector molecule, in proper orientation and proximity to expression control elements resident in the expression vector molecule so as to direct expression of one or more proteins when the vector molecule resides in an appropriate host cell.

Construction of appropriate expression vectors and other recombinant or genetic modification techniques for practising the invention are well known in the art (see, *e.g.,* Green and Sambrook, Molecular Cloning, A Laboratory Manual, Cold Spring Harbor Laboratory Press (Cold Spring Harbor, N.Y.) (2012), and Ausubel et al., Short Protocols in Molecular Biology, Current Protocols John Wiley and Sons (New Jersey) (2002), and references cited herein). Appropriate microbial cells and vectors are available commercially through, for example, the American Type Culture Collection (ATCC), Rockville, Md.

The term "host cell" refers to any cell into which an exogenous nucleic acid sequence can be introduced and expressed, typically via an expression vector. The host cell may, for example, be a wild-type cell isolated from its natural environment, a mutant cell identified by screening, a cell of a commercially available strain, or a genetically engineered cell or mutant cell, comprising one or more other exogenous and/or heterologous nucleic acid sequences than those of the invention.

A "recombinant" cell or host cell as used herein refers to a host cell into which one or more exogenous nucleic acid sequences of the invention have been introduced, typically via transformation of a host cell with a vector.

The term "substrate" or "precursor", as used herein in relation to a specific enzyme, refers to a molecule upon which the enzyme acts to form a product. When used in relation to an exogenous biometabolic pathway, the term "substrate" or "precursor" refers to the molecule(s) upon which the first enzyme of the referenced pathway acts, such as, e.g., GTP in the pathway shown in **Figure 1****.** When referring to an enzyme-catalyzed reaction in a microbial cell, an "endogenous" substrate or precursor is a molecule which is native to or biosynthesized by the microbial cell, whereas an "exogenous" substrate or precursor is a molecule which is added to the microbial cell, via a medium or the like. For example, in the biometabolic pathway shown in **Figure 1****,** GTP is normally present in microbial host cells, and is therefore an endogenous substrate.

Unless otherwise stated, the term "sequence identity" for amino acid sequences as used herein refers to the sequence identity calculated as *(n_{ref}* - *n_{dif}*)·100/*n_{ref},* wherein *n_{dif}* is the total number of non-identical residues in the two sequences when aligned and wherein *n_{ref}* is the number of residues in one of the sequences. Hence, the amino acid sequence GSTDYTQNWA will have a sequence identity of 80% with the sequence GSTGYTQAWA (*n_{dif}*=2 and *n_{ref}*=10). The sequence identity can be determined by conventional methods, *e.g.,* Smith and Waterman, (1981), Adv. Appl. Math. 2:482, by the 'search for similarity' method of Pearson & Lipman, (1988), Proc. Natl. Acad. Sci. USA 85:2444, using the CLUSTAL W algorithm of Thompson et al., (1994), Nucleic Acids Res 22:467380, by computerized implementations of these algorithms (GAP, BESTFIT, FASTA, and TFASTA in the Wisconsin Genetics Software Package, Genetics Computer Group). The BLAST algorithm (Altschul et al., (1990), Mol. Biol. 215:403-10) for which software may be obtained through the National Center for Biotechnology Information www.ncbi.nlm.nih.gov/) may also be used. When using any of the aforementioned algorithms, the default parameters for "Window" length, gap penalty, etc., are used. Preferably, the sequence identity between two amino acid sequences is determined using the Needleman-Wunsch algorithm (Needleman and Wunsch, 1970, J. Mol. Biol. 48: 443- 453) as implemented in the Needle program of the EMBOSS package (EMBOSS: The European Molecular Biology Open Software Suite, Rice et al., 2000, Trends Genet. 16: 276- 277), preferably version 5.0.0 or later, *e.g.,* as provided at World-Wide Web-address ebi.ac.uk/Tools/psa/emboss_needle/. The parameters used are typically a gap open penalty of 10, a gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows: (*Identical Residues* × *100)*/*(Length of Alignment* - *Total Number of Gaps in Alignment*)*.* In some embodiments, sequence identity values as used herein do not take into account specifically identified mutations of the embodiment it refers to, *e.g.,* specifically identified amino acid substitutions.

Enzymes referred to herein can be classified on the basis of the handbook Enzyme Nomenclature from NC-IUBMB, 1992), see also the ENZYME site at the internet: http://www.expasy.ch/enzyme/. This is a repository of information relative to the nomenclature of enzymes, and is primarily based on the recommendations of the Nomenclature Committee of the International Union of Biochemistry and Molecular Biology (IUB-MB). It describes each type of characterized enzyme for which an EC (Enzyme Commission) number has been provided (Bairoch A., The ENZYME database, 2000, Nucleic Acids Res 28:304-305). The IUBMB Enzyme nomenclature is based on the substrate specificity and occasionally on their molecular mechanism.

### Specific embodiments of the invention

As indicated in the Summary, the present invention relates to variants of TPH having improved tryptophan hydroxylase activity over the native parent enzyme and to variants of GCH1 (FolE) providing for improved amino acid hydroxylase activity in a microbial cell. The present invention also relates to recombinant microbial cells comprising a heterologous monooxygenase, a heterologous PCD and, optionally, a heterologous and/or variant GCH1.

### Monooxygenases

As used herein, suitable monooxygenases include, but are not limited to, phenylalanine hydroxylase (EC 1.14.16.1), phenylalanine 3-hydroxylase (EC 1.14.16.7), tyrosine 3-hydroxylase (EC 1.14.16.2), anthranilate 3-monooxygenase (EC 1.14.16.3), mandelate 4-monooxygenase (EC 1.14.16.6) and alkylglycerol monooxygenase (EC 1.14.16.5). Preferably, the monooxygenase is (a) capable of catalyzing the addition of a hydroxyl-group to an aromatic ring, preferably the aromatic ring of an aromatic amino acid such as, *e.g.,* tryptophan, tyrosine, phenylalanine, histidine, thyroxine, 5HTP and/or L-DOPA, preferably aromatic amino acids in L-form; (b) capable of utilizing one or more of MH4, THB, tetra hydrofolate, 5-methyl-tetra hydrofolate, 5-formyl-tetrahydrofolate, 10-formyl-tetrahydrofolate, 6-carboxy-tetrahydropterin, and 6-hydroxymethyl-tetrahydropterin as cofactor for the referenced reaction. Particularly preferred are amino acid hydroxylases, with aromatic amino acid hydroxylases such as TPHs, THs and PheHs being most preferred. In a particular embodiment, the aromatic amino acid hydroxylase is not a PheH from a non-flowering plant.

Suitable TPHs, THs and PheHs include those listed in Table 1.

### 1. L-Tryptophan hydroxylase (TPH)

Sources of nucleic acid sequences encoding a TPH include any species where the encoded gene product is capable of catalyzing the referenced reaction, including humans, mammals such as, *e.g.,* mouse, cow, horse, chicken and pig, as well as other animals such as, *e.g.,* the parasite *Schistosoma mansoni.* In humans and, it is believed, in other mammals, there are two distinct TPH alleles, referred to herein as TPH1 and TPH2, respectively. As used herein, a TPH is an enzyme capable of catalyzing the hydroxylation of L-tryptophan to 5HTP.

Examples of nucleic acids encoding TPH for use in recombinant cells according to aspects and embodiments of the present invention include, but are not limited to, those encoding the TPHs listed in **Table 1,** as well as functionally active variants, homologs and fragments thereof. The amino acid sequence of a TPH from *Mesocricetus auratus* (Golden hamster) is provided in NCBI Reference Sequence NP_001297486.1.

Functional fragments and variants of TPH enzymes are known in the art. For example, to increase heterologous expression in *E. coli* and the enzyme stability, the TPH sequence can be truncated to remove portions not needed for its catalytic activity which preserving the catalytic core of the enzyme. Specific examples of functional fragments of TPH include Met102 to Ser416 of *Oryctolagus cuniculus* TPH (Moran et al., J Biol Chem 1998;273(20): 12259-66) and residues Asp45-Arg471 or Glu147-Thr460 (*i.e*., E147 to T460) of *Homo sapiens* TPH2, optionally comprising an added N-terminal methionine residue (see SEQ ID NOS: 12 and 13, respectively). Other TPH sequences can be similarly truncated to create functionally active fragments or variants comprising the catalytic core. For example, SEQ ID NO: 11 represents a fragment of *Homo sapiens* TPH2 comprising an added heterologous 20-amino acid polypeptide at its C-terminal. The *Homo sapiens* TPH2 sequence reported as NCBI accession No. NP_775489 (SEQ ID NO: 10) has a 6-amino acid insert in the N-terminal portion.

Notably, *Schistosoma mansoni* TPH (SEQ ID NO:9) has advantageous properties with respect to, e.g., solubility, thus enabling no or less truncation of the enzyme sequence. In addition, as reported in Example 3, *Schistosoma mansoni* TPH has advantageous catalytic activity in an optimized recombinant cell according to the invention. Accordingly, in one embodiment, the TPH is *Schistosoma mansoni* TPH, or a functionally active fragment and/or variant thereof.

In one embodiment of the recombinant cell according to any aspect of the invention, the TPH is a mammalian TPH, such as, *e.g., Homo sapiens* TPH2, or a fragment and/or variant thereof. In a preferred embodiment, the TPH comprises or consists essentially of a fragment of a TPH which corresponds to (*i.e*., aligns with) residues E147 to T460 or L148 to T460 of *Homo sapiens* TPH2 (SEQ ID NO:3), typically with an N-terminal methionine (M) residue; or a catalytically active variant thereof. This particular hsTPH fragment is set forth as SEQ ID NO:13.

In one embodiment, the TPH or TPH fragment is a variant comprising one or more mutations, such as insertions, deletions or amino acid substitutions, in at least one residue as compared to the reference (native parent) sequence. Preferably, a TPH variant has a mutation in a residue corresponding to a residue selected from E147 (if present), Asp242 (N, asparagine) and Pro244 (P, proline) in *Homo sapiens* TPH2.

The mutation in residue corresponding to E147 is preferably an amino acid substitution to a Lys (K; lysine), Arg (R; arginine) or His (H; histidine), most preferably an 147K mutation. Even more preferably, the nucleic acid sequence encoding the TPH has the 5'-end sequence *atgaaa,* encoding MK. In a specific embodiment, the nucleic acid sequence is operatively linked to a Trc promoter, optionally comprising the nucleic acid sequence of SEQ ID NO:53. Without being limited to theory, the mutation encoding for the N-terminal Met-Lys residues provides for an increased activity and/or expression level of the TPH.

The mutation in the residue corresponding to residue N242 in *Homo sapiens* TPH2 (SEQ ID NO:3) is preferably an amino acid substitution to Ile (I; isoleucine), *i.e*., 242I, whereas the mutation in residue P244 is preferably an amino acid substitution to a Cys (C, cysteine), Asp (D, aspartic acid), Leu (L, leucine) or Glu (Q, glutamine), *i.e*., 244C, 244D, 244L or 244Q, preferably 244D or 244C, such as 244D. The variant may also comprise a combination of two or more amino acid substitutions, such as, *e.g.,* 242I/244D or 242I/244C, optionally in combination with 147K.

In a particularly preferred embodiment, the TPH variant comprises or consists of the amino acid sequence of SEQ ID NO: 13 with E2K, N97I and P99C mutations introduced.

In another aspect, the invention provides SEQ ID NO: 13 having E2K, N97I and P99C mutations.

Typically, the TPH variant has tryptophan hydroxylation activity and a sequence identity of at least 30%, such as at least 50%, such as at least 60%, such as at least 70%, such as at least 80%, such as at least 90%, such as at least 91%, 92%, 93%, 94%, 95%, 96%, 97% or 98%, such as at least 99%, over at least the catalytically active portion, optionally over the full length, of the native TPH amino acid sequence in its mature form, excluding signal peptide sequences and the like. Preferably, a TPH variant comprising one or more mutations according to the invention provides for a tryptophan hydroxylation activity at least similar, typically higher than of the reference TPH, typically the native or parent TPH. For example, the TPH variant may provide for a tryptophan hydroxylation activity which is at least about at least about 100%, such as at least about 110%, such as at least about 130%, such as at least about 150%, such as at least about 200%, such as at least 250% of that of the native or parent TPH. Since TPH may also have phenylalanine hydroxylation activity, a TPH variant comprising one or more mutations according to the invention may also or alternatively provide for a phenylalanine hydroxylation activity at least similar to, or higher than, that of the reference TPH, typically the native or parent TPH. For example, the TPH variant may provide for a phenylalanine hydroxylation activity which is at least about 90%, such as at least about 100%, such as at least about 110%, such as at least about 130%, such as at least about 150%, such as at least about 200%, such as at leats 250%, of that of the native or parent TPH, *e.g., Homo sapiens* TPH2.

Assays for measuring TPH activity *in vitro* are well-known in the art (see, *e.g.,* Winge et al., Biochem J, 2008;410:195-204 and Moran *et al.,* 1998). Suitable assays are also provided by the present Examples, *e.g.,* the assays in Examples 2-4, reflecting the ability of the TPH to catalyze the conversion of L-tryptophan to 5HTP, and Examples 6 and 7, reflecting the ability of the TPH to catalyze the conversion of L-phenylalanine to L-tyrosine.

In the recombinant host cell, the TPH is typically sufficiently expressed so that an increased level of 5HTP production from L-tryptophan can be detected as compared to the microbial host cell prior to transformation with the TPH, optionally in the presence of added THB cofactor and/or tryptophan substrate. Typically, in the context of the present invention, THB cofactor is not added. Alternatively, the expression level of the specific TPH enzyme can be evaluated by proteomic analysis, according to methods known in the art. In a preferred embodiment, the nucleic acid sequence encoding the TPH is operably linked to a strong promoter such as the Trc promoter, providing for high expression levels of the TPH.

### 2. L-Tyrosine hydroxylase (TH)

Sources of nucleic acid sequences encoding a TH include any species where the encoded gene product is capable of catalyzing the referenced reaction, including humans, mammals such as, *e.g.,* mouse, cow, horse, chicken and pig, as well as other animals. As used herein, a TH is an enzyme capable of catalyzing the hydroxylation of L-tyrosine to L-DOPA.

Examples of nucleic acids encoding a TH for use in aspects and embodiments of the recombinant cell according to the present invention include, but are not limited to, those encoding the THs listed in **Table 1,** as well as catalytically active variants, homologs and fragments thereof. Exemplary variants and fragments of a TH include those resulting in the N-terminal sequence MK, *e.g.,* by making an X2K amino acid substitution in a native TH having the N-terminal sequence MX (Met-Xaa), wherein X is not K (Lys), or an insertion of a K between the N-terminal M and X amino acid residues, In one embodiment, the TH is *Rattus norwegicus* TH, or a functionally active variant, homolog or fragment thereof.

Assays for measuring TH activity *in vitro* are well-known in the art. A suitable assay is also provided in the present Example 5, reflecting the ability of the TH to catalyze the conversion of L-tyrosine to L-3,4-dihydroxyphenylalaine (L-DOPA).

In the recombinant host cell, the TH is typically sufficiently expressed so that an increased level of L-DOPA production from L-tyrosine can be detected as compared to the microbial host cell prior to transformation with the TH, optionally in the presence of added THB cofactor and/or tyrosine substrate. Typically, in the context of the present invention, THB cofactor is not added. Alternatively, the expression level of the specific TH enzyme can be evaluated by proteomic analysis, according to methods known in the art. In a preferred embodiment, the nucleic acid sequence encoding the TH is operably linked to a strong promoter such as the Trc promoter, providing for high expression levels of the TH.

### 3. L-Phenylalanine hydroxylase (PheH)

Sources of nucleic acid sequences encoding a PheH include any species where the encoded gene product is capable of catalyzing the referenced reaction, including humans, mammals such as, e.g., mouse, cow, horse, chicken and pig, as well as other animals. As used herein, a PheH is an enzyme capable of catalyzing the hydroxylation of L-phenylalanine to tyrosine.

Examples of nucleic acids encoding a PheH for use in aspects and embodiments of the recombinant cell according to the present invention include, but are not limited to, those encoding the PheHs listed in **Table 1,** as well as functionally active variants, homologs and fragments thereof. Exemplary variants and fragments of a PheH include those resulting in the N-terminal sequence MK, *e.g.,* by making an X2K amino acid substitution in a native PheH having the N-terminal sequence MX, wherein X is not K, or an insertion of a K between the N-terminal M and X amino acid residues, In one embodiment, the PheH is *C*. *violaceum* PheH, or a functionally active variant, homolog or fragment thereof.

In a specific embodiment, the PheH is a phenylalanine 3-hydroxylase, catalyzing the conversion of phenylalanine to m-tyrosine.

Assays for measuring PheH activity *in vitro* are well-known in the art. A suitable assay is also provided in the present Example 1, reflecting the ability of the PheH to catalyze the conversion of L-phenylalanine to L-tyrosine, as well as Examples 6 and 7.

In the recombinant host cell, the PheH is typically sufficiently expressed so that an increased level of L-tyrosine (or a variant thereof, such as, *e.g.,* L-m-tyrosine) production from L-phenylalanine can be detected as compared to the microbial host cell prior to transformation with the PheH, optionally in the presence of added THB cofactor and/or phenylalanine substrate. Typically, in the context of the present invention, THB cofactor is not added. Alternatively, the expression level of the specific PheH enzyme can be evaluated by proteomic analysis, according to methods known in the art. In a preferred embodiment, the nucleic acid sequence encoding the PheH is operably linked to a strong promoter such as the Trc promoter, providing for high expression levels of the PheH.

### Pterin-4a-carbolamine dehydratase (PCD)

The PCD is typically classified as EC 4.2.1.96, and converts HTHB to DHB in the presence of water, as shown in Figure 1. Exemplary nucleic acids encoding PCD enzymes for use in aspects and embodiments of the recombinant cell according to the present invention include, but are not limited to, those encoding the PCDs shown in **Table 1,** as well as functionally active variants, homologs and fragments thereof. In other embodiments, the exogenous nucleic acid encoding a PCD can encode a PCD which is endogenous to the microbial host cell, *e.g.,* in the case of host cells from *Chromobacterium violaceum, Bacillus cereus, Lactobacillus ruminis Pseudomonas aeruginosa* or *Rhodobacteraceae* bacterium. In some embodiments, the microbial host cell endogenously comprises a sufficient amount of a native PCD. In such cases, transformation of the host cell with an exogenous nucleic acid encoding a PCD is optional.

Exemplary variants and fragments of a PCD include those resulting in the N-terminal sequence MK, *e.g.,* by making an X2K amino acid substitution in a native PCD having the N-terminal sequence MX, wherein X is not K, or an insertion of a K between the N-terminal M and X amino acid residues, In one embodiment, the PCD is C. *violaceum* PheH, or a functionally active variant, homolog or fragment thereof. In a preferred embodiment of the recombinant cell according to any aspect of the invention, the PCD comprises or consists essentially of *Chromobacterium violaceum* PCD (SEQ ID NO:21) or a functionally active variant, homolog or fragment thereof.

Assays for measuring PCD activity *in vitro* are well-known in the art (see, *e.g.,* Köster et al., Biol. Chem. 1998; 379: 1427-1432). In the recombinant host cell, a sufficient expression of the PCD can typically be detected using an indirect assay, for example, measuring phenylalanine to tyrosine conversion of a PheH or TPH in a tyrosine auxotroph (see, *e.g.,* Examples 1, 6 and 7). The expression level of the specific PCD enzyme can be evaluated according to methods known in the art, e.g., by proteomic analysis. In a preferred embodiment, the nucleic acid sequence encoding the PCD is operably linked to a strong promoter, providing for high expression levels of the PCD.

### GTP cyclohydrolase I (GCH1)

Sources of nucleic acid sequences encoding a GCH1 include any species where the encoded gene product is capable of catalyzing the referenced reaction. Examples of nucleic acids encoding a GCH1 for use in recombinant cells according to aspects and embodiments of the present invention include, but are not limited to, those encoding the GCH1s listed in **Table 1,** as well as functionally active variants, homologs and fragments thereof. As used herein, a GCH1 is an enzyme capable of catalyzing the conversion of GTP to 7,8-dihydroneopterin triphosphate.

In one embodiment, the recombinant microbial cell comprises a GCH1 having the same or similar catalytic activity as *E. coli* GCH1 (FolE; SEQ ID NO: 16) or another GCH1 listed in **Table 1,** or a catalytically active fragment and/or variant thereof. Advantageously, the GCH1 is FolE or a variant of FolE. FolE is a homodecameric protein and is composed of a pentamer of five dimers, and is the first enzyme in several biosynthetic pathways for production of tetrahydrofolates, tetrahydromonapterin and others which may act as monooxygenase (e.g., TPH, TH and PheH) cofactors in *E. coli* (see **Figure 1**).

Surprisingly, as described in Examples 1 and 4-7, it was found that mutations in a number of residues of *E. coli* GCH1 were capable of increasing the supply of a cofactor for TPH, PheH and TH in *E. coli* cells. Accordingly, in one aspect of the invention, there is provided a variant of *E. coli* FolE (SEQ ID NO: 16) comprising one or more mutations, wherein, in an *E. coli* cell comprising a pterin-4a-carbinolamine dehydratase (PCD) and at least one of a tryptophan hydroxylase (TPH), a tyrosine hydroxylase (TH) and a phenylalanine hydroxylase (PheH), the variant provides for an increased hydroxylation activity of at least one of the TPH, TH and PheH as compared to native E. coli GCH1. In one embodiment, the mutation is not T198P. The amino acid sequence of the variant is characterized by at least 94% sequence identity to the native FolE sequence, as described below.

In one embodiment, at least one of the one or more mutations is in an amino acid residue in a segment selected from D97-E112, K121-D130, N170-H180, S193-L200 and S207-N222 which is D97, M99, T101, V102, A125, K129, N170, V179, T196, S199, L200, S207, H212, E213, F214, L215, H221, or a combination thereof. Preferred, non-limiting amino acid residues for such mutations include, but are not limited to, D97, M99, N170, V179, L200, S207, H212, E213, F214, L215, and H221. Most preferably, the amino acid residue is at least one amino acid residue selected from M99, V179, T198, L200, F214, and L215. In one embodiment, the amino acid sequence of the variant is characterized by at least 98% sequence identity to the native FolE sequence and at least one of the one or more mutations is in T198, wherein the mutation in T198 is not T198P.

In one embodiment, exemplary amino acid substitutions in these segments include, but are not limited to, D97V, D97L, D97A, D97T, M99C, M99T, M99V, M99L, M99I, T101I, T101V, T101L, V102M, N170K, N170D, N170L, V179A, V179M, T196I, T196V, T196L, T198I, T198V, T198S, T198L, S199Y, S199F, L200P, L200C, L200S, L200A, S207R, S207K, S207M, H212R, H212K, E213K, E213R, F214A, F214G, F214S, L215P, L215Q, L215N, L215D, L215T, L215S, L215G, L215A, L215C, L215F, L215M, H221R, H221K, and combinations thereof. Preferred, non-limiting, mutations in these amino acid residues include D97V, M99I, N170K, N170D, V179A, V179M, T198I, T198S, L200P, S207R, H212R, E213K, F214S, L215Q, L215P and H221R, and combinations of any two or more thereof. In separate and specific embodiments, the variant comprises a mutation selected from M99I, V179A, T198I, T198S, L200P, F214S, and L215P.

In another embodiment, exemplary amino acid residues for further mutations include, without limitation: S3, S5, H12, A14, V28, H29, A41, G42, E46, N52, D57, M61, E62, I67, A68, I75, D97, M99, V102, T108, T117, A125, K129, I133, Q157, N170, V179, K184, G187, T198, S199, L200, S207, H212, E213, F214, L215, H221, N222, and combinations thereof. Examples of amino acid substitutions in these residues include, without limitation, S3L, S5C, H12R, A14V, V28L, V28A, H29Y, A41G, G42D, E46D, N52K, D57V, M61I, E62K, I67V, A68S, I75V, D97V, M99I, V102M, T108N, T117I, A125D, K129N, I133F, Q157L, N170K, N170D, V179A, V179M, K184R, G187S, T198I, T198S, S199Y, L200P, S207R, H212R, E213K, F214S, L215P, L215Q, H221R, N222I, and combinations thereof.

Specific, non-limiting combinations of mutations include:
(a) I67V, T117I, A125D and H221R
(b) E62K, N170K and L215P
(c) V102M and L215P
(d) Q157L and H212R
(e) V28L, L215P and N222I
(f) T108N, I133F and E213K
(g) S5C, D57V and L215Q
(h) H29Y, I75V and V179M
(i) A14V, E46D, M61I and D97V
(j) V28A, G42D and E213K
(k) N52K, A68S and S207R
(l) A41G, K129N and I133F
(m) S3L, K184R and S199Y; and
(n) H12R, N170D and G187S.

Nar *et al.* (1995) and Rebelo *et al.* (2003) have reported on the structure of FolE. The T198 residue is located in the 4^{th} β-strand that is located in close proximity to an α-helix defined by Ser207 to Asn222, apparently involved in oligomerization of the FolE complex. Thus, T198 can play a role in FolE oligomerization. Specifically, the mutation could affect the hydrophobic interaction with Leu215 and subsequently the positioning of the Ser207-Asn222 helix and overall polymerization of the FolE complex. Moreover, the alignment in Figure 4 indicates that several of these segments conserved in the *Homo sapiens* (SEQ ID NO: 14) and S. *cerevisiae* (SEQ ID NO:17) homologs of FolE.

So, in another specific embodiment, there is provided a functionally active variant of *E. coli* GCH1 (FolE; SEQ ID NO:16) comprising a mutation in the residue corresponding to D97, e.g., a D97V, D97L, D97A or D97T substitution. In one embodiment, the GCH1 is *E. coli* GCH1 wherein, preferably, the mutation is D97V.

In another specific embodiment, there is provided a functionally active variant of *E. coli* GCH1 (FolE; SEQ ID NO: 16) comprising a mutation in the residue corresponding to M99, e.g., a M99C, M99T, M99V, M99L or M99I substitution. In one embodiment, the GCH1 is *E. coli* GCH1 wherein, preferably, the mutation is M99I.

In another specific embodiment, there is provided a functionally active variant of *E. coli* GCH1 (FolE) comprising a mutation in the residue corresponding to N170, *e.g.,* a N170K, N170D or N170L substitution. In one embodiment, the GCH1 is *E. coli* GCH1, wherein, preferably, the mutation is N170K or N170D.

In another specific embodiment, there is provided a functionally active variant of *E. coli* GCH1 (FolE; SEQ ID NO: 16) comprising a mutation in the residue corresponding to V179, *e.g.,* a V179A or V179M substitution. In one embodiment, the GCH1 is *E. coli* GCH1 wherein, preferably, the mutation is V179A.

In a specific embodiment, there is provided a functionally active variant, homolog or fragment of *E. coli* GCH1 (FolE; SEQ ID NO: 16) comprising a mutation in the residue corresponding to T198, *e.g.,* a T198S, T198I, T198V or T198L substitution, wherein the T198 substitution is not T198P. In one embodiment, the GCH1 is *E. coli* GCH1 wherein, preferably, the mutation is T198S or T198I.

In another specific embodiment, there is provided a functionally active variant of *E. coli* GCH1 (FolE; SEQ ID NO:16) comprising a mutation in the residue corresponding to L200, *e.g.,* a L200P, L200C, L200S or L200A substitution. In one embodiment, the GCH1 is *E. coli* GCH1 wherein, preferably, the mutation is L200P.

In another specific embodiment, there is provided a functionally active variant of *E. coli* GCH1 (FolE; SEQ ID NO:16) comprising a mutation in the residue corresponding to S207, *e.g.,* a S207R, S207K, or S207M substitution. In one embodiment, the GCH1 is *E. coli* GCH1 wherein, preferably, the mutation is S207R.

In another specific embodiment, there is provided a functionally active variant of *E. coli* GCH1 (FolE; SEQ ID NO:16) comprising a mutation in the residue corresponding to H212, *e.g.,* a H212R or H212K substitution. In one embodiment, the GCH1 is *E. coli* GCH1 wherein, preferably, the mutation is H212R.

In another specific embodiment, there is provided a functionally active variant of *E. coli* GCH1 (FolE; SEQ ID NO:16) comprising a mutation in the residue corresponding to E213, *e.g.,* a E213K or E213R substitution. In one embodiment, the GCH1 is *E. coli* GCH1 wherein, preferably, the mutation is E213K.

In another specific embodiment, there is provided a functionally active variant of *E. coli* GCH1 (FolE; SEQ ID NO:16) comprising a mutation in the residue corresponding to F214, *e.g.,* a F214A, F214G or F214S substitution. In one embodiment, the GCH1 is *E. coli* GCH1 wherein, preferably, the mutation is F214S.

In another specific embodiment, there is provided a functionally active variant of *E. coli* GCH1 (FolE; SEQ ID NO:16) comprising a mutation in the residue corresponding to L215, *e.g.,* a L215P, L215Q, L215N, L215D, L215T, L215S, L215G, L215A, L215C, L215F or L215M substitution. In one embodiment, the GCH1 is *E. coli* GCH1 wherein, preferably, the mutation is L215P or L215Q.

In another specific embodiment, there is provided a functionally active variant of *E. coli* GCH1 (FolE; SEQ ID NO:16) comprising a mutation in the residue corresponding to H221, *e.g.,* a H221R or H221K substitution. Preferably, the mutation in FolE is H221R.

In a specific embodiment, a GCH1 variant of any preceding embodiment comprises the native, mature GCH1 sequence except for the listed mutation(s).

In one embodiment, any *E. coli* GCH1 (FolE) variant or fragment retain the native residues corresponding to C111, C182, H113 and H114 of the enzyme since, as reported by Rebelo (2003), replacement of these residues made the enzyme catalytically inactive.

In another aspect, the invention provides FolE (SEQ ID NO: 16) having a mutation selected from D97V, M99I, N170K, N170D, V179A, V179M, T198I, T198S, L200P, S207R, H212R, E213K, F214S, L215Q, L215P and H221R, or a combination of any two or more thereof.

The functionally active GCH1 variant has a sequence identity of at least 94%, 95%, 96%, 97% or 98%, such as at least 99% to native *E. coli* GCH1 having the sequence of SEQ ID NO: 16.

The GCH1 variants of the invention may provide for one, two or all of a tryptophan, tyrosine and phenylalanine hydroxylation activity which is at least similar to, typically higher than than that of the reference GCH1, e.g., the native or parent GCH1. This can be investigated in a microbial cell comprising the GCH1 variant, the hydroxylase and a PCD.

In one embodiment, a GCH1 variant comprising mutations in one or more specific amino acid residues or one or more specific amino acid substitutions according to the invention provides for a tryptophan hydroxylation activity at least similar to, typically higher than that of the reference GCH1, typically the native or parent GCH1, in a microbial cell comprising a TPH. For example, such a GCH1 variant may, in an *E. coli* cell comprising a TPH and a PCD provide for a tryptophan hydroxylase activity, converting L-tryptophan to 5HTP, which is at least about 100%, such as at least about 110%, such as at least about 130%, such as at least about 150%, such as at least about 200%, such as at least about 250%, such as at least about 300% of that of the native or parent GCH1. Suitable assays are provided in the Examples, e.g., Example 4. For example, the TPH may be *Homo sapiens* TPH2 (SEQ ID NO:3) or a functionally active variant, homolog or fragment thereof, such as a TPH variant comprising or consisting of the amino acid sequence of SEQ ID NO: 13 with E2K, N97I and P99C mutations introduced; the PCD may, for example, be C. *violaceum* PCD (SEQ ID NO:21) or a functionally active variant, homolog or fragment thereof; and the assay conducted in the presence of about 100 mg/L L-tryptophan.

In one embodiment, a GCH1 variant comprising mutations in one or more specific amino acid residues or one or more specific amino acid substitutions according to the invention provides for a tyrosine hydroxylation activity at least similar to, typically higher than that of the reference GCH1, typically the native or parent GCH1, in a microbial cell comprising a TH. For example, such a GCH1 variant may, in an *E. coli* cell comprising a TH and a PCD provide for a tyrosine hydroxylase activity, converting L-tyrosine to L-DOPA, which is at least about 100%, such as at least about 110%, such as at least about 130%, such as at least about 150%, such as at least about 200%, such as at least about 250%, such as at least about 300% of that of the native or parent GCH1. Suitable assays are provided in the Examples, e.g., Example 5. For example, the TPH may be *Rattus norvegicus* TH (SEQ IDS NO:52) or a functionally active variant, homolog or fragment thereof; the PCD may, for example, be C. *violaceum* PCD (SEQ ID NO:21) or a functionally active variant, homolog or fragment thereof, and the assay conducted in the presence of about 100 mg/L L-tyrosine.

In one embodiment, a GCH1 variant comprising mutations in one or more specific amino acid residues or one or more specific amino acid substitutions according to the invention provides for a phenylalanine hydroxylation activity at least similar to, typically higher than that of the reference GCH1, typically the native or parent GCH1, in a microbial cell comprising a PheH. For example, such a GCH1 variant may, in an *E. coli* cell comprising a PheH (which may also be a TPH having phenylalanine hydroxylation activity), and a PCD provide for a phenylalanine hydroxylase activity, converting L-phenylalanine to tyrosine, which is at least about 100%, such as at least about 110%, such as at least about 130%, such as at least about 150%, such as at least about 200%, such as at least about 250%, such as at least about 300% of that of the native or parent GCH1. Suitable assays are provided in the Examples, e.g., Examples 1, 6 and 7. For example, the PheH may be C. *violaceum* PheH (SEQ ID NO:58) or a functionally active variant, homolog or fragment thereof; the PCD may, for example, be C. *violaceum* PCD (SEQ ID NO:21) or a functionally active variant, homolog or fragment thereof, and the assay conducted in the presence of about 100 mg/L L-phenylalanine. Alternatively, the PheH may be a TPH also having PheH activity, such as *Homo sapiens* TPH2 (SEQ ID NO:3) or a functionally active variant, homolog or fragment thereof, such as a TPH variant comprising or consisting of the amino acid sequence of SEQ ID NO: 13 with E2K, N97I and P99C mutations introduced.

A mutated GCH1 according to any aspect or embodiment herein can, for example, be expressed from an exogenously introduced nucleic acid sequence. In one embodiment, the mutated GCH1 is expressed from a nucleic acid sequence integrated into the chromosome. Alternatively, the native gene can be mutated *in situ, i.e.,* in the chromosome of the microbial cell, after which the mutated GCH1 is expressed from the mutated gene. In some embodiments, the microbial cell expresses a lower level of native GCH1 than the (native) parent cell, *e.g.,* by downregulating, deleting or otherwise inactivating the native GCH1 gene (*e.g., folE*).

In a preferred embodiment, the nucleic acid sequence encoding the GCH1 is operably linked to a strong promoter, providing for high expression levels of the GCH1. Even more preferably, the nucleic acid sequence encoding the GCH1 has the 5'-end sequence *atgaaa,* encoding MK. In a specific embodiment, the nucleic acid sequence is operatively linked to a Trc promoter, optionally comprising the nucleic acid sequence of SEQ ID NO:53. Without being limited to theory, the mutation encoding for the N-terminal Met-Lys residues provides for an increased activity and/or expression level of the GCH1.

In the recombinant host cell, the GCH1 is typically sufficiently expressed so that an increased level of 5HTP production from L-tryptophan can be detected in the assay according to Example 2, Example 3 or Example 4, *i.e.,* in an *E. coli* host cell comprising a *Homo sapiens* TPH2 fragment or variant as described herein or a *Schistosoma mansoni* TPH, and a C. *violaceum* PCD, as compared to the microbial host cell prior to transformation with a heterologous GCH1 or prior to the introduction of a GCH1, optionally in the presence of added tryptophan substrate. Alternatively, the expression level of the specific GCH1 enzyme can be evaluated by proteomic analysis, according to methods known in the art.

### Downstream enzymes

In some embodiments, particularly when the monooxygenase is a TH, the recombinant microbial cell can further comprise nucleic acid sequences, *e.g.,* heterologous, encoding a dopa decarboxylase, a tyramine oxidase and an alcohol dehydrogenase for production of hydroxytyrosol; *e.g.,* as described by Satoh et al. (2012).

In some embodiments, particularly when the monooxygenase is a TPH and serotonin, N-acetyl-serotonin or melatonin is a desired end-product, the recombinant microbial cell can further comprise heterologous nucleic acid sequences encoding a 5HTP decarboxylase (ADDC), a serotonin acetyltransferase (AANAT) and/or an acetylserotonin O-methyltransferase (ASMT):

### 1. 5HTP decarboxylase:

The last step in the serotonin biosynthesis via a 5HTP intermediate, the conversion of 5HTP to serotonin, is in animal cells catalyzed by a 5HTP decarboxylase, which is an aromatic L-amino acid decarboxylase (AADC) typically classified as EC 4.1.1.28. Suitable 5HTP decarboxylases include any tryptophan decarboxylase (TDC) capable of catalyzing the referenced reaction. TDC participates in the plant serotonin biosynthesis pathway, where tryptophan decarboxylase (TDC) catalyzes the conversion of tryptophan to tryptamine, which is then converted into serotonin in a reaction catalyzed by tryptamine 5-hydroxylase (T5H). TDC likewise belongs to the aromatic amino acid decarboxylases categorized in EC 4.1.1.28, and can be able to convert 5HTP to serotonin and carbon dioxide (see, *e.g.,* Park et al., Biosci. Biotechnol. Biocem. 2008;72(9):2456-2458.2008, and Gibson et al., J. Exp. Bot. 1972;23(3):775-786), and thus function as a 5HTP decarboxylase. Exemplary nucleic acids encoding ADDC enzymes for use in aspects and embodiments of the present invention include, but are not limited to, those encoding the 5HTP decarboxylases listed in **Table 1,** as well as functionally active variants, homologs and fragments thereof. In some embodiments, particularly where it is desired to also promote serotonin formation from a tryptamine substrate in the same recombinant cell, an enzyme capable of catalyzing both the conversion of tryptophan to tryptamine and the conversion of 5HTP to serotonin can be used. For example, rice TDC and tomato TDC can function also as a 5HTP decarboxylase, an activity which can be promoted by the presence of pyridoxal phosphate (*e.g.,* at a concentration of about 0.1 mM) (Park *et al.,* 2008).

Suitable assays for testing serotonin production by a 5HTP decarboxylase in a recombinant microbial host cell are provided in, or can be adapted from, *e.g.,* Park *et al.* (2008) and Park et al., Appl Microbiol Biotechnol 2011;89(5):1387-1394. For example, these assays can be adapted to test serotonin production by a 5HTP decarboxylase (*e.g.,* a TDC), either from 5HTP or, in case the microbial cell comprises an L-tryptophan hydroxylase, from L-tryptophan (or simply a carbon source). In one exemplary embodiment, the recombinant microbial cell produces at least 5%, such as at least 10%, such as at least 20%, such as at least 50%, such as at least 100% or more serotonin than the recombinant cell without transformation with 5HTP decarboxylase enzyme, *i.e*., a background value.

### 2. Serotonin acetyltransferase (AANAT):

In one aspect, the recombinant microbial cell further comprises an exogenous nucleic acid sequence encoding a serotonin acetyltransferase, also known as serotonin -N-acetyltransferase, arylalkylamine N-acetyltransferase and AANAT, and typically classified as EC 2.3.1.87. AANAT catalyzes the conversion of acetyl-CoA and serotonin to CoA and N-Acetyl-serotonin. Exemplary nucleic acids encoding AANAT enzymes for use in aspects and embodiments of the present invention include, but are not limited to, those encoding the AANATs shown in **Table 1,** as well as functionally active variants, homologs or fragments thereof. Suitable assays for testing N-acetylserotonin production by an AANAT in a recombinant microbial host cell are described in, *e.g.,* Thomas et al., Analytical Biochemistry 1990;184:228-34.

### 3. Acetylserotonin O-methyltransferase (ASMT):

In one aspect, the recombinant cell further comprises an exogenous nucleic acid encoding an acetylserotonin O-methyltransferase or ASMT, typically classified as EC 2.1.1.4. ASMT catalyzes the last reaction in the production of melatonin from L-tryptophan, the conversion of N-acetyl-serotonin and S-adenosyl-L-methionine (SAM) to Melatonin and S-adenosyl-L-homocysteine (SAH). SAH can then be recycled back to SAM via the S-adenosyl-L-methionine cycle in microbial cells where the S-adenosyl-L-methionine cycle is native (or exogenously added) and constitutively expressed, such as, *e.g.,* in *E.coli.* Exemplary nucleic acids encoding ASMT enzymes for use in aspects and embodiments of the present invention include, but are not limited to, those encoding ASMTs shown in **Table 1,** as well as functionally active variants, homologs or fragments thereof. Suitable assays for testing melatonin production by an ASMT in a recombinant microbial host cell have been described in, *e.g.,* Kang et al. J. Pineal Res. 2011:50;304-309.

### Nucleic acids and vectors

In other aspects, there is provided nucleic acid sequences and vectors comprising such nucleic acid sequences. For example, a nucleic acid sequence encoding an enzyme or other protein activity listed in **Table 1** or a fragment or variant thereof as described in any aspect or embodiment herein may encode an amino acid sequence that is homologous (*i.e*., native) or heterologous to the recombinant host cell in question, *e.g.,* a variant or fragment of an endogenous amino acid sequence.

Also provided are one or more vectors comprising nucleic acid sequences according to the above aspects and embodiments, *e.g.,* encoding one or more of, such as one, two, three, four, five, six or all of, a monooxygenase (*e.g.,TPH*/*TH*/*PheH),* PCD, GCH1, 5HTP decarboxylase, AANAT and ASMT, optionally wherein the GCH1 is mutant FolE as described herein and/or the TPH is *Schistosoma mansoni* or variant *Homo sapiens* TPH2 as described herein.

A nucleic acid sequence may comprise a Trc promoter and a DNA coding sequence encoding an enzyme or other protein, wherein the 3'-end of the Trc promoter is operably, preferably directly, linked to the 5'-end of the DNA coding sequence, and wherein the 5'-end sequence of the DNA coding sequence is *atgaaa.*

A vector may comprise such a nucleic acid sequence.

A method for expressing an enzyme or other protein may comprise transforming an *E*. *coli* host cell with the vector of the preceding embodiment, expressing the enzyme or other protein; and, optionally, harvesting the enzyme or other protein.

Preferably, the Trc promoter is directly linked to the DNA coding sequence. Most preferably, the nucleic acid sequence comprises SEQ ID NO:53, i.e., the DNA sequence of a Trc promoter directly linked to *atgaaa.* The N-terminal amino acids of the enzyme or other protein encoded by the DNA coding sequence are MK (Met-Lys). Accordingly, the enzyme or other protein encoded by the DNA coding sequence may be a native enzyme or protein whose N-terminal amino acid sequence is MK. Alternatively, the enzyme or other protein may be a variant or fragment of a native enzyme or protein, wherein the N-terminal amino acid sequence is modified to MK, *e.g.,* introducing an X2K amino acid substitution, where "X" represents the amino acid adjacent to the N-terminal methionine residue. The enzyme encoded may be one that is listed in **Table 1** or a functionally active fragment or variant thereof, wherein the N-terminal amino acids are MK.

The specific design of the vector according to any aspect or embodiment depends on, *e.g.,* whether host cell already endogenously produces sufficient amounts of one or more of the enzymes. For example, in an *E*. *coli* host cell, it may not be necessary to introduce the nucleic acid sequence encoding a mutated FolE sequence exogenously, in case the native gene can be mutated *in situ* to introduce the mutation (see Example 1). Additionally, for transformation of a particular host cell, two or more vectors with different combinations of the enzymes used in the present invention can be applied. The vector can be a plasmid, phage vector, viral vector, episome, an artificial chromosome or other polynucleotide construct, and may, for example, include one or more selectable marker genes and appropriate expression control sequences.

Generally, regulatory control sequences are operably linked to the encoding nucleic acid sequences, and include constitutive, regulatory and inducible promoters, transcription enhancers, transcription terminators, and the like which are well known in the art. The encoding nucleic acid sequences can be operationally linked to one common expression control sequence or linked to different expression control sequences, such as one inducible promoter and one constitutive promoter.

The procedures used to ligate the various regulatory control and marker elements with the encoding nucleic acid sequences to construct the vectors of the present invention are well known to one skilled in the art (see, *e.g.,* Sambrook *et al.,* 2012, supra). In addition, methods have recently been developed for assembling of multiple overlapping DNA molecules (Gibson *et al.,* 2008) (Gibson *et al.,* 2009) (Li & Elledge, 2007), allowing, *e.g.,* for the assembly multiple overlapping DNA fragments by the concerted action of an exonuclease, a DNA polymerase and a DNA ligase.

The promoter sequence is typically one that is recognized by the intended host cell. For an *E.coli* host cell, suitable promoters include, but are not limited to, the lac promoter, the T7 promoter, pBAD, the tet promoter, the Lac promoter, the Trc promoter, the Trp promoter, the recA promoter, the λ (lamda) promoter, and the PL promoter. Preferred promoters include the Trc promoter. For *Streptomyces* host cells, suitable promoters include that of *Streptomyces coelicolor agarase* (dagA). For a Bacillus host cell, suitable promoters include the sacB, amyL, amyM, amyQ, penP, xylA and xylB. Other promoters for bacterial cells include prokaryotic beta-lactamase (Villa-Kamaroff et al., 1978, Proceedings of the National Academy of Sciences USA 75: 3727-3731), and the tac promoter (DeBoer et al., 1983, Proceedings of the National Academy of Sciences USA 80: 21-25). For an S. cerevisiae host cell, useful promoters include the TEF1, HXT7, TDH3, ENO-1, GAL1, ADH1, ADH2, GAP, TPI, CUP1, PHO5 and PGK, such as PGK1 promoters. Other useful promoters for yeast host cells are described by Romanos et al., 1992, Yeast 8: 423-488. Still other useful promoters for various host cells are described in "Useful proteins from recombinant bacteria" in Scientific American, 1980, 242: 74-94; and in Sambrook *et al.,* 2012, *supra.*

In one embodiment, one or more or all of the exogenous nucleic acids is each under the control of a strong promoter, *e.g.,* each separately selected from T7, lac, trac and PL in an *E. coli* host cell, and each separately selected from PGK1, TEF1, HXT7 and TDH3 in an S. *cerevisiae* host cell.

A transcription terminator sequence is a sequence recognized by a host cell to terminate transcription, and is typically operably linked to the 3' terminus of an encoding nucleic acid sequence. Suitable terminator sequences for *E. coli* host cells include the T7 terminator region. Suitable terminator sequences for yeast host cells such as S. *cerevisiae* include CYC1, PGK, GAL, ADH, AOX1 and GAPDH. Other useful terminators for yeast host cells are described by Romanos *et al.,* 1992, *supra.*

A leader sequence is a non-translated region of an mRNA which is important for translation by the host cell. The leader sequence is typically operably linked to the 5' terminus of a coding nucleic acid sequence. Suitable leaders for yeast host cells include S. cerevisiae ENO-1, PGK, alpha-factor, ADH2/GAP, TEF, and Kozak sequence.

A polyadenylation sequence is a sequence operably linked to the 3' terminus of a coding nucleic acid sequence which, when transcribed, is recognized by the host cell as a signal to add polyadenosine residues to transcribed mRNA. Useful polyadenylation sequences for yeast host cells are described by Guo and Sherman, 1995, Mol Cell Biol 15: 5983-5990.

A signal peptide sequence encodes an amino acid sequence linked to the amino terminus of an encoded amino acid sequence, and directs the encoded amino acid sequence into the cell's secretory pathway. In some cases, the 5' end of the coding nucleic acid sequence may inherently contain a signal peptide coding region naturally linked in translation reading frame, while a foreign signal peptide coding region may be required in other cases. Useful signal peptides for yeast host cells can be obtained from the genes for S. *cerevisiae* alpha-factor and invertase. Other useful signal peptide coding regions are described by Romanos *et al.,* 1992, supra. An exemplary signal peptide for an *E. coli* host cell can be obtained from alkaline phosphatase. For a *Bacillus* host cell, suitable signal peptide sequences can be obtained from alpha-amylase and subtilisin. Further signal peptides are described by Simonen and Palva, 1993, Microbiological Reviews 57: 109-137.

It may also be desirable to add regulatory sequences which allow the regulation of the expression of the polypeptide relative to the growth of the host cell. Examples of regulatory systems are those which cause the expression of the gene to be turned on or off in response to a chemical or physical stimulus, including the presence of a regulatory compound. Regulatory systems in prokaryotic systems include the lac, tec, and tip operator systems. For example, one or more promoter sequences can be under the control of an IPTG inducer, initiating expression of the gene once IPTG is added. In yeast, the ADH2 system or GAL1 system may be used. Other examples of regulatory sequences are those which allow for gene amplification. In eukaryotic systems, these include the dihydrofolate reductase gene which is amplified in the presence of methotrexate, and the metallothionein genes which are amplified with heavy metals. In these cases, the respective encoding nucleic acid sequence would be operably linked with the regulatory sequence.

The choice of the vector will typically depend on the compatibility of the vector with the host cell into which the vector is to be introduced. The vectors may be linear or closed circular plasmids. The vector may also be an autonomously replicating vector, *i.e*., a vector which exists as an extrachromosomal entity, the replication of which is independent of chromosomal replication, *e.g.,* a plasmid, an extrachromosomal element, a minichromosome, or an artificial chromosome. The vector may contain any means for assuring self-replication. Alternatively, the vector may be one which, when introduced into the host cell, is integrated into the genome and replicated together with the chromosome(s) into which it has been integrated. Furthermore, a single vector or plasmid or two or more vectors or plasmids which together contain the total DNA to be introduced into the genome of the host cell, or a transposon may be used.

The vectors of the present invention preferably contain one or more selectable markers which permit easy selection of transformed cells. The selectable marker genes can, for example, provide resistance to antibiotics or toxins, complement auxotrophic deficiencies, or supply critical nutrients not in the culture media, and/or provide for control of chromosomal integration. Examples of bacterial selectable markers are the dal genes from Bacillus subtilis or Bacillus licheniformis, or markers which confer antibiotic resistance such as ampicillin, kanamycin, chloramphenicol, or tetracycline resistance. Suitable markers for yeast host cells are ADE2, HIS3, LEU2, LYS2, MET3, TRP1, and URA3.

The vectors of the present invention may also contain one or more elements that permit integration of the vector into the host cell genome or autonomous replication of the vector in the cell independent of the genome. For integration into the host cell genome, the vector may rely on an encoding nucleic acid sequence or other element of the vector for integration into the genome by homologous or nonhomologous recombination. Alternatively, the vector may contain additional nucleotide sequences for directing integration by homologous recombination into the genome of the host cell at a precise location(s) in the chromosome(s). To increase the likelihood of integration at a precise location, the integrational elements should preferably contain a sufficient number of nucleic acids, such as 100 to 10,000 base pairs, preferably 400 to 10,000 base pairs, and most preferably 800 to 10,000 base pairs, which have a high degree of identity with the corresponding target sequence to enhance the probability of homologous recombination. The integrational elements may be any sequence that is homologous with the target sequence in the genome of the host cell. The integrational elements may, for example, non-encoding or encoding nucleotide sequences. The vector may be integrated into the genome of the host cell by non-homologous recombination. For example, for integration into an *E. coli* chromosome, the vector may contain elements directing integration of the nucleic acid sequences using the Tn7 site-specific integration method according to McKenzie G and Nancy LC (2006).

For autonomous replication, the vector may further comprise an origin of replication enabling the vector to replicate autonomously in the host cell in question. The origin of replication may be any plasmid replicator mediating autonomous replication which functions in a cell. The term "origin of replication" or "plasmid replicator" is defined herein as a nucleotide sequence that enables a plasmid or vector to replicate *in vivo.* Examples of bacterial origins of replication are the origins of replication of plasmids pBR322, pUC19, pACYC177, and pACYC184 permitting replication in *E. coli,* and pUB1 10, pE194, pTA1060, and pAMβi permitting replication in Bacillus. Examples of origins of replication for use in a yeast host cell are the 2 micron origin of replication, ARS1, ARS4, the combination of ARS1 and CEN3, and the combination of ARS4 and CEN6.

More than one copy of the nucleic acid sequence encoding the enzyme or protein of interest may be inserted into the host cell to increase production of the gene product. An increase in the copy number of the encoding nucleic acid sequence can be obtained by integrating at least one additional copy of the sequence into the host cell genome or by including an amplifiable selectable marker gene with the nucleic acid sequence where cells containing amplified copies of the selectable marker gene, and thereby additional copies of the sequence, can be selected for by cultivating the cells in the presence of the appropriate selectable agent.

### Recombinant cells

The present invention relates to recombinant microbial cells comprising a monooxygenase and a PCD. Optionally, one or both enzymes are heterologous to the microbial cell. It has now been discovered that the production of 5HTP, L-DOPA, tyrosine (*e.g.,* m-tyrosine) and other compounds that are the products of monooxygenase-catalyzed reactions in such recombinant microbial cells can be optimized by modifications such as omitting certain enzymes and/or introducing certain mutations in certain endogenous or exogenous nucleic acid sequences. These modifications include mutations in the monooxygenase (*e.g.,* a TPH) or in a GTP cyclohydrolase 1 (GCH1) as described in previous embodiments, and/or mutations in YnbB, mutations, downregulation or deletion of tnaA,and combinations of any two or more such modifications.

The aforementioned mutations can advantageously be combined in the recombinant host cell, particularly when the host cell is of the *Escherichia* genus, such as an *E. coli* cell. Any monooxygenase, *e.g.,* an amino acid hydroxylase, can be used. In some embodiments, however, the monooxygenase is a TPH, a TH or a PheH. So, in one embodiment, the recombinant microbial cell comprises a PCD and one or more of (a) a nucleic acid sequence encoding *E. coli* GTP cyclohydrolase 1 (folE) or a functionally active variant, homolog or fragment thereof, comprising a mutation as described herein, *e.g.,* in T198, F214, V179, M99 and/or L200; and (b) a nucleic acid sequence encoding a mammalian (such as a *Homo sapiens)* TPH or *Schistosoma mansoni* TPH or a functionally active variant, homolog or fragment thereof. In preferred embodiments, the mutation in FolE is selected from T198I, T198S, F214S, V179A, M99I and L200P and/or the TPH comprises or consists of the residues corresponding to residues E147 to T460 of *Homo sapiens* TPH2, an N-terminal methionine residue, and a mutation corresponding to E147K, N242I or P244D/P244C or a combination thereof such as N242I and P244D.

In one embodiment, the recombinant microbial cell, particularly when the cell is an *E. coli* cell, also or alternatively comprises an YnbB or a functionally active variant, homolog or fragment thereof, comprising a mutation in the residue corresponding to V197. Preferably, the mutation in V197 is V197A. The *E. coli* YnbB sequence is provided in SEQ ID NO:52.

In one embodiment, particularly when the monooxygenase is a TPH, the recombinant microbial cell of the invention comprises a deletion or downregulation of an endogenous gene encoding an enzyme providing for degradation of tryptophan, *e.g.,* a tryptophanase (EC 4.1.99.1).

In one embodiment, the microbial cell is genetically modified, typically mutated, to downregulate or delete tryptophanase activity. Tryptophanase or tryptophan indole-lyase (EC 4.1.99.1), encoded by the *tnaA* gene in *E. coli,* catalyzes the hydrolytic cleavage of L-tryptophan to indole, pyruvate and NH4⁺. Active tryptophanase consists of four identical subunits, and enables utilization of L-tryptophan as sole source of nitrogen or carbon for growth together with a tryptophan transporter encoded by tnaC gene. Tryptophanase is a major contributor towards the cellular L-cysteine desulfhydrase (CD) activity. *In vitro,* tryptophanase also catalyzes o, β elimination, β replacement, and α hydrogen exchange reactions with a variety of L-amino acids. Tryptophan degradation mechanisms are known to also exist in other microorganisms. For instance, in *S*. *cerevisiae,* there are two different pathways for the degradation of tryptophan (The Erlich pathway and the kynurenine pathway, respectively), involving in their first step the aromatic amino acid aminotransferase ARO8, ARO9, ARO10, and/or BNA2 genes.

Accordingly, reducing tryptophan degradation can be achieved by, *e.g.,* a site-directed mutation in or deletion of a gene encoding a tryptophanase, such as the tnaA gene (in *E. coli* or other organisms such as *Enterobacter aerogenes*) (Uniprot P0A853), or the kynA gene (in Bacillus species) (Uniprot Q736W5), or one or more of the ARO8 (Uniprot P53090), ARO9 (Uniprot P38840), ARO10 (Uniprot Q06408)and BNA2 (Uniprot P47125) genes (in S. *cerevisiae*)*.* In one embodiment, the ARO9 gene is downregulated, optionally deleted. Alternatively, tryptophanase activity can be reduced reducing the expression of the gene by introducing a mutation in, *e.g.,* a native promoter element, or by adding an inhibitor of the tryptophanase.

In addition, as discovered by the present inventor, for the purpose of achieving 5HTP production in a microbial cell, it is not necessary that the cell comprises exogenous nucleic acid sequences providing for the expression of dihydropteridine reductase (DHPR) and/or dihydromonapterin reductase (DHMR). It is further not necessary for the cell to comprise exogenous nucleic sequences providing for the expression of 6-pyruvoyl-tetrahydropterin synthase (PTPS or PTS) and/or sepiapterin reductase (SPR). Without being limited to theory, this provides for increased metabolic resources being available for the expression of TPH and PCD, the production of 5HTP production, or both, thus improving the yield of 5HTP.

DHPR is typically classified as EC 1.5.1.34, and can convert quinonoid dihydrobiopterin (q-H₂-BPt or DHB) to tetrahydrobiopterin (H₄-BPt or THB) in the presence of cofactor NADH. DHMR is typically classified as EC 1.5.1.50 and can convert 7,8-dihydromonapterin to 5,6,7,8-tetrahydromonapterin. The endogenous *E. coli* DHMR gene is designated *foIM.*So*,* in one embodiment, the recombinant microbial cell of any one of the preceding aspects or embodiments does not comprise one or both of a heterologous or overexpressed endogenous DHPR and DHMR. In a specific embodiment, the microbial cell does not comprise an exogenous nucleic acid sequence, such as a heterologous or overexpressed endogenous nucleic acid sequence, encoding a DHPR or a DHMR. In one embodiment, the microbial cell does not comprise a nucleic acid encoding a DHPR or a DHMR. In an additional or alternative embodiment, the microbial cell does not comprise a nucleic acid sequence encoding *E. coli* oxygen-insensitive nitroreductase (NfsB).

PTS is typically classified as EC 4.2.3.12, and can convert 7,8-dihydroneopterin 3'-triphosphate ((H₂-N₃P or DHP) to 6-pyruvoyltetrahydropterin (H₄-PPt or 6PTH). SPR is typically classified as EC 1.1.1.153, and can convert 6PTH to THB in the presence of its cofactor NADPH.

So, in one embodiment, the recombinant microbial cell of any preceding aspect or embodiment does not comprise one or both of a heterologous or overexpressed endogenous PTPS and SPR. In a specific embodiment, the microbial cell does not comprise an exogenous nucleic acid sequence, such as a heterologous or overexpressed endogenous nucleic acid sequence, encoding a PTS or an SPR. In another specific embodiment, the microbial cell does not comprise a nucleic acid sequence encoding a PTS or an SPR.

In yet another embodiment, the recombinant microbial cell of any preceding aspect or embodiment does not comprise any heterologous or overexpressed endogenous DHPR, DHMR, PTPS or SPR. In a specific embodiment, the microbial cell does not comprise exogenous nucleic acid sequences, such as heterologous or overexpressed endogenous nucleic acid sequences, encoding a DHPR, a DHMR, PTS or SPR. In another specific embodiment, the microbial cell does not comprise nucleic acid sequences encoding a DHPR, a DHMR, a PTS or an SPR.

**Table 1 - Examples of enzymes and amino acid sequences**

| **Name (EC** #) | **Species** | **SEQ ID # (GenBank or UniProtKB #)** |
|---|---|---|
| L-tryptophan hydroxylase (EC 1.14.16.4) (TPH) | *Oryctolagus cuniculus TPH1 ("ocTPH")* | 1 (P17290-1, v2) |
| | *Homo sapiens TPH1 ("hsTPH1")* | 2 (NP_004170.1) |
| | *Homo sapiens TPH2 ("hsTPH2")* | 3 (NP_775489.2, Q8IWU9) |
| | *Bos taurus ("bsTPH")* | 4 |
| | *Sus scrofa ("scTPH")* | 5 |
| | *Gallus gallus ("ggTPH")* | 6 (NP_990287.1) |
| | *Mus musculus ("mmTPH")* | 7 (NP_033440.1) |
| | *Equus caballus ("ecTPH")* | 8 (NP_001075252.1) |
| | *Schistosoma mansoni ("scTPH")* | 9 (AAD01923.1) |
| | *Homo sapiens TPH2, insert (+6)* | 10 |
| | *Homo sapiens TPH2, truncated ((45-471)*+*20)* | 11 |
| | *Homo sapiens TPH2, truncated (45-471)* | 12 |
| | *Homo sapiens TPH2, truncated (147-460+N-terminal methionine)* | 13 |
| GTP cyclohydrolase I (EC 3.5.4.16) (GCH1) | *Homo sapiens* | 14 (P30793) |
| | *Mus musculus* | 15 (Q3U7P6) |
| | *E. coli (FoIE)* | 16 (P0A6T5) |
| | *S. cerevisiae* | 17 (P51601) |
| | *Bacillus subtilis* | 18 (G4EUF8) |
| | *Streptomyces avermitilis* | 19 (Q82EE8) |
| | *Salmonella typhii* | 20 (T2PZ12) |
| pterin-4-alpha-carbinolamine dehydratase (EC 4.2.1.96) (PCD) | *Chromobacterium violaceum* | 21 (Q7NVH7) |
| | *Pseudomonas aeruginosa* | 22 (P43335) |
| | *Bacillus cereus* var. *anthracis* | 23 (D8GWB2) |
| | *Lactobacillus ruminis* ATCC 25644 | 25 (E7FT68) |
| | *Rhodobacteraceae bacterium* HTCC2083 | 26 (B6B2H7) |
| | *Homo sapiens* | 27 (P61457) |
| 5HTP decarboxylase (EC 4.1.1.28) (ADDC) | *Acidobacterium capsulatum* | 28 (WP_015898075.1) |
| | *Rattus norwegicus* | 29 (XP_006251536.1) |
| | *Sus scrofa* | 30 (NP_999019.1) |
| | *Homo sapiens* | 31 (P20711-1, v2) |
| | *Capsicum annuum* | 32 (NP_001312016.1) |
| | *Drosophila caribiana* | 33 (AAM80956.1) |
| | *Maricaulis maris* (strain MCS10) | 34 (ABI65701.1) |
| | *Oryza sativa* subsp. Japonica | 35 (XP_015648768.1) |
| | *Pseudomonas putida* S16 | 36 (WP_013972057.1) |
| | *Catharanthus roseus* | 37 (P17770-1, v1) |
| serotonin acetyltransferase (EC 2.3.1.87 or 2.3.1.5) (AANAT) | *Chlamydomonas reinhardtii* | 38 (BAH10512.1) |
| | *Bos Taurus* | 39 (DAA18183.1) |
| | *Bos Taurus A55P* | 40 |
| | *Gallus gallus* | 41 (NP_990489.1) |
| | *Homo sapiens* | 42 (NP_001079.1) |
| | *Mus musculus* | 43 (XP _011246971.1) |
| | *Oryctolagus cuniculus* | 44 (XP_008249128.1) |
| | *Ovis aries* | 45 (NP_001009461.1) |
| acetylserotonin O-methyltransferase (EC 2.1.1.4) (ASMT) | *Oryza sativa* | 46 (XP_015610997.1) |
| | *Homo sapiens* | 47 (P46597-1, v1) |
| | *Bos Taurus* | 48 (P10950-1, v2) |
| | *Rattus norvegicus* | 49 (NP_653360.2) |
| | *Gallus gallus* | 50 (NP_990674.1) |
| | *Macaca mulatta* | 51 (NP_001028112.1) |
| | *Ocimum basilicum* | Q9XGV9-1, v1 |
| | *Takifugu rubripes* | (XP_011609423.1) |
| | *Elephantulus edwardii* | (XP_006902482.1) |
| | *Chromobacterium violaceum* | (WP_011135808.1) |
| | *Desulfotomaculum kuznetsovii DSM 6115* | (YP_004515712.1) |
| | *Xenopus (Silurana) tropicalis* | (NP_001011409.1) |
| | *Pseudomonas fluorescens* | (WP_019095725.1) |
| | *Candidatus Solibacter usitatus* | (WP_011682595.1) |
| | *Fenneropenaeus chinensis* | (AAZ66373.1) |
| | *Arabidopsis thaliana* | (NP_200227.1) |
| Tyrosine hydroxylase (TH) (EC 1.14.16.2) | *Rattus norwegicus* | 52 (NP_036872.1) |
| | *Homo sapiens* | 54 (P07101) |
| | *Mus musculus* | 55 (P24529) |
| | *Bos taurus* | 56 (P17289) |
| | *Gallus gallus* | 57 (Q9PU40) |
| Phenylalanine hydroxylase (PheH) (EC 1.14.16.1, EC 1.14.16.7) | *Chromobacterium violaceum* | 58 (P30967) |
| | *Xanthomonas campestris pv. Viticola* | 59 (A0A077SF23) |
| | *Pseudomonas aeruginosa* | 60 (P43334) |
| | *Pseudomonas putida* | 61 (Q6EMJ5) |
| | *Homo sapiens* | 62 (P00439) |
| | *Mus musculus* | 63 (P16331) |
| | *Streptomyces coeruleorubidus* | 64 (F5BFC8) |

Variants or homologs of any one or more of the enzymes and other proteins listed in **Table 1,** having the referenced activity and a sequence identity of at least 30%, such as at least 50%, such as at least 60%, such as at least 70%, such as at least 80%, such as at least 90%, such as at least 91%, 92%, 93%, 94%, 95%, 96%, 97% or 98%, such as at least 99%, over at least the catalytically active portion, optionally over the full length, of the reference amino acid sequence in its mature form, excluding signal peptide sequences and the like, are also contemplated. The variant or homolog may comprise, for example, 2, 3, 4, 5 or more, such as 10 or more, amino acid substitutions, insertions or deletions as compared to the reference amino acid sequence in its mature form. In particular conservative substitutions are considered. These are typically within the group of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions which do not generally alter specific activity are known in the art and are described, for example, by H. Neurath and R. L. Hill, 1979, In: The Proteins, Academic Press, New York. The most commonly occurring exchanges are Ala to Ser, Val to Ile, Asp to Glu, Thr to Ser, Ala to Gly, Ala to Thr, Ser to Asn, Ala to Val, Ser to Gly, Tyr to Phe, Ala to Pro, Lys to Arg, Asp to Asn, Leu to Ile, Leu to Val, Ala to Glu, and Asp to Gly. Homologs, such as orthologs or paralogs, having the desired activity can be identified in the same or a related animal or microbial species using the reference sequences provided and appropriate activity testing. Specific enzyme variants are exemplified herein.

In a particularly preferred embodiment, the variant of an amino acid sequence in **Table 1** has an X2K amino acid substitution, where "X" represents the amino acid adjacent to the N-terminal methionine residue.

The recombinant host cell is typically prepared by introducing, typically via transformation, one or more vectors as described herein, using standard methods known in the art (see, e.g., Sambrook *et al.,* 2012, supra). The introduction of a vector into a bacterial host cell may, for instance, be effected by protoplast transformation (see, e.g., Chang and Cohen, 1979, Molecular General Genetics 168: 111-115), using competent cells (see, *e.g*., Young and Spizizen, 1961 , Journal of Bacteriology 81 : 823-829, or Dubnau and Davidoff-Abelson, 1971, Journal of Molecular Biology 56: 209-221 ), electroporation (see, *e.g*., Shigekawa and Dower, 1988, Biotechniques 6: 742-751), or conjugation (see, *e.g*., Koehler and Thome, 1987, Journal of Bacteriology 169: 5771-5278).

As described above, the vector, once introduced, may be maintained as a chromosomal integrant or as a self-replicating extra-chromosomal vector.

Preferably, for transformation of an *E. coli* or other bacterial host cell, the vectors are designed as follows: A *lac* promoter is used to control the expressions of a gene or an artificial operon containing up to three genes connected with a linker sequence, in order to express the genes at a suitable level so that the introduction of heterologous genes/pathways do not overdraw substrates or energy in the host cell. In one particular embodiment, the recombinant microbial cell, preferably a bacterial cell, is transformed according to a strategy outlined in the Examples.

Preferably, for transformation of a yeast host cell such as S. *cerevisiae,* the heterologous genes are integrated onto chromosome using a homologous recombination based method (Mikkelsen *et al.,* 2012). As compared with gene expression based on plasmids, the chromosomal integrated genes can be expressed with higher fidelity and resulted in better protein translation, in particular for multiple gene co-expression systems.

The transformation can be confirmed using methods well known in the art. Such methods include, for example, nucleic acid analysis such as Northern blots or polymerase chain reaction (PCR) amplification of mRNA, or immunoblotting for expression of gene products, or other suitable analytical methods to test the expression of an introduced nucleic acid sequence or its corresponding gene product, including those referred to above and relating to measurement of 5HTP production. Expression levels can further be optimized to obtain sufficient expression using methods well known in the art and as disclosed herein.

Tryptophan, tyrosine and phenylalanine production takes place in all known microorganisms by a single metabolic pathway (Somerville, R. L., Herrmann, R. M., 1983, Amino acids, Biosynthesis and Genetic Regulation, Addison-Wesley Publishing Company, U.S.A.: 301-322 and 351-378; Aida et al., 1986, Bio-technology of amino acid production, progress in industrial microbiology, Vol. 24, Elsevier Science Publishers, Amsterdam: 188-206). The recombinant microbial cell of the invention can thus be prepared from any microbial host cell, using recombinant techniques well known in the art (see, *e.g*., Sambrook *et al.,* 2012, supra, and Ausubel *et al.* (1991), supra. Preferably, the host cell is tryptophan, tyrosine and/or phenylalanine autotrophic (*i.e*., capable of endogenous biosynthesis of the substrate of the oxidation reaction of interest), grows on synthetic medium with suitable carbon sources, and expresses a suitable RNA polymerase (such as, *e.g.,* T7 polymerase).

GTP cyclohydrolase I (such as, *e.g*. FolE) -catalyzed pterin biosynthesis takes place in many organisms including both prokaryotes and eukaryotes. The recombinant cell of the invention can thus be prepared from any hosts, using recombinant techniques well known in the art (see, *e.g*., Sambrook *et al.,* 2012, supra, and Ausubel *et al.* (1991), supra. Preferably, the host cell is capable of one, more or all of tetrahydrofolate, tetrahydrobiopterin, preQ₀, drosopterin, aurodrosopterin or tetrahydromonapterin biosynthesis (see **Figure 1**)**.** So, for example, in one embodiment, the microbial host cell is an *E. coli* cell comprising the endogenous enzymes foIE, foIX, P-ase, and foIM, optionally upregulated or expressed from one or more vectors.

For embodiments where the monooxygenase is a TPH and the desired end-product is serotonin, N-acetylserotonin or melatonin, the recombinant host cell is typically capable of biosynthesizing and/or regenerating the cofactors used by the enzymes in the melatonin biosynthesis pathway. In particular, the recombinant host cell is preferably capable of biosynthesizing, regenerating, or bio-synthesizing and regenerating, one or more cofactors for TPH, AANAT and ASMT. Most types of host cells (*e.g*., mammalian host cells, yeast host cells such as *S*. *cerevisiae,* bacteria such as *E. coli,* etc.) are capable of producing and regenerating acetyl-CoA and SAM; the cofactors for AANAT and ASMT, respectively. AcCoA serves as a metabolic cofactor in the AANAT reaction, but is also part of other, endogenous pathways in, *e.g*., microbial cells.

SAM is a principal methyl donor in various intracellular transmethylation reactions. It is synthesized in the cell through SAM synthetase from methionine and ATP, and natively generated through the SAM cycle, which consists of a methyl transferase, an S-adenosyl-L-homocysteine hydrolase, a folate transferase, and an S-adenosyl-methionine synthetase (Lee et al., Korean J. Chem. Eng. 2010, 27, 587-589). Accordingly, in the ASMT-catalyzed, last reaction in the production of melatonin from L-tryptophan, N-acetylserotonin and SAM are converted to melatonin and SAH. SAH can then be recycled back to SAM via the SAM-cycle in microbial cells where the S-adenosyl-L-methionine cycle is native (or exogenously added) and constitutively expressed, such as, *e.g*., in *E.coli.* The enzymes of such native pathways can also, in needed, be upregulated or expressed from an exogenously introduced vector, using well-known recombinant techniques known in text books referenced elsewhere herein. Non-limiting and exemplary nucleic acids encoding enzymes of the SAM cycle for use in recombinant cells according to aspects and embodiments of the present invention include those shown in Table 1 of WO 2015/032911 A1, including the actual amino acid sequences referred to in the table as SEQ ID numbers.

The microbial host cell for use in the present invention is typically unicellular and can be, for example, a bacterial cell, a yeast host cell, a filamentous fungal cell, or an algeal cell. Examples of suitable host cell genera include, but are not limited to, *Acinetobacter, Agrobacterium, Alcaligenes, Anabaena, Aspergillus, Bacillus, Bifidobacterium, Brevibacterium, Candida, Chlorobium, Chromatium, Corynebacteria, Cytophaga, Deinococcus, Enterococcus, Erwinia, Erythrobacter, Escherichia, Flavobacterium, Hansenula, Klebsiella, Lactobacillus, Methanobacterium, Methylobacter, Methylococcus, Methylocystis, Methylomicrobium, Methylomonas, Methylosinus, Mycobacterium, Myxococcus, Pantoea, Phaffia, Pichia, Pseudomonas, Rhodobacter, Rhodococcus, Saccharomyces, Salmonella, Sphingomonas, Streptococcus, Streptomyces, Synechococcus, Synechocystis, Thiobacillus, Trichoderma, Yarrowia and Zymomonas.*

In one embodiment, the host cell is bacterial cell, *e.g.,* an *Escherichia* cell such as an *Escherichia coli* cell; a *Bacillus* cell such as a *Bacillus alkalophilus, Bacillus amyloliquefaciens, Bacillus brevis, Bacillus circulans, Bacillus clausii, Bacillus coagulans, Bacillus lautus, Bacillus lentus, Bacillus licheniformis, Bacillus megaterium, Bacillus stearothermophilus, Bacillus subtilis,* or a *Bacillus thuringiensis* cell; or a *Streptomyces* cell such as a *Streptomyces lividans* or *Streptomyces murinus* cell. In a particular embodiment, the host cell is an *E*. *coli* cell. In another particular embodiment, the host cell is of an *E*. *coli* strain selected from the group consisting of K12.DH1 (Proc. Natl. Acad. Sci. USA, volume 60, 160 (1968)), JM101, JM103 (Nucleic Acids Research (1981), 9, 309), JA221 (J. Mol. Biol. (1978), 120, 517), HB101 (J. Mol. Biol. (1969), 41, 459) and C600 (Genetics, (1954), 39, 440).

In one embodiment, the host cell is a fungal cell, such as, *e.g.,* a yeast cell. Exemplary yeast cells include *Candida, Hansenula, Kluyveromyces, Pichia, Saccharomyces, Schizosaccharomyces and Yarrowia* cells. In a particular embodiment, the host cell is an *S. cerevisiae* cell. In another particular embodiment, the host cell is of an *S*. *cerevisie* strain selected from the group consisting of *S. cerevisiae* KA31, AH22, AH22R-, NA87-11A, DKD-5D and 20B-12, *S*. *pombe* NCYC1913 and NCYC2036 and *Pichia pastoris* KM71.

The recombinant microbial cell of any one of the aspects or embodiments herein is typically a bacterial cell, a yeast cell, a filamentous fungal cell, or an algal cell. In one embodiment, the recombinant microbial cell is a cell of the *Escherichia* genus, such as an *Escherichia coli* cell. In one embodiment, the recombinant microbial is derived from a *Saccharomyces,* a *Schizosaccharomyces,* a *Corynebacterium,* a *Bacillus* or a *Streptomyces* cell.

### Production of oxidation products and downstream products:

The invention also provides a method of producing various oxidation products of an aromatic amino acid. Examples of products include, but are not limited to, 5HTP, L-DOPA, tyrosine (*e.g.,* m-tyrosine), hydroxytyrosol, melatonin, serotonin and/or N-acetyl-serotonin, comprising culturing the recombinant microbial cell of any preceding aspect or embodiment in a medium comprising a carbon source. The desired compound can then optionally be isolated or retrieved from the medium, and optionally further purified. Importantly, using a recombinant microbial cell according to the invention, the method can be carried out without adding monooxygenase substrates such as L-tryptophan, L-tyrosine or L-phenylalanine, or monooxygenase cofactor such as THB, or both, to the medium.

Also provided is a method of preparing a composition comprising one or more compounds selected from 5HTP, L-DOPA, tyrosine, hydroxytyrosol, melatonin, serotonin and/or N-acetyl-serotonin, comprising culturing the recombinant microbial cell of any preceding aspect or embodiment, isolating and purifying the compound(s), and adding any excipients to obtain the composition.

Suitable carbon sources include carbohydrates such as monosaccharides, oligosaccharides and polysaccharides. As used herein, "monosaccharide" denotes a single unit of the general chemical formula Cx(H2O)y, without glycosidic connection to other such units, and includes glucose, fructose, xylose, arabinose, galactose and mannose. "Oligosaccharides" are compounds in which monosaccharide units are joined by glycosidic linkages, and include sucrose and lactose. According to the number of units, oligosacchardies are called disaccharides, trisaccharides, tetrasaccharides, pentasaccharides etc. The borderline with polysaccharides cannot be drawn strictly; however the term "oligosaccharide" is commonly used to refer to a defined structure as opposed to a polymer of unspecified length or a homologous mixture. "Polysaccharides" is the name given to a macromolecule consisting of a large number of monosaccharide residues joined to each other by glycosidic linkages, and includes starch, lignocellulose, cellulose, hemicellulose, glycogen, xylan, glucuronoxylan, arabinoxylan, arabinogalactan, glucomannan, xyloglucan, and galactomannan. Other suitable carbon sources include acetate, glycerol, pyruvate and gluconate. In one embodiment, the carbon source is selected from the group consisting of glucose, fructose, sucrose, xylose, mannose, galactose, rhamnose, arabinose, fatty acids, glycerine, glycerol, acetate, pyruvate, gluconate, starch, glycogen, amylopectin, amylose, cellulose, cellulose acetate, cellulose nitrate, hemicellulose, xylan, glucuronoxylan, arabinoxylan, glucomannan, xyloglucan, lignin, and lignocellulose. In one embodiment, the carbon source comprises one or more of lignocellulose and glycerol. In one embodiment, the carbon source is a simple carbon source such as glucose, xylose, fructose, arabinose, galactose, mannose, glycerol, acetate, or a mixture of any thereof.

The culture conditions are adapted to the recombinant microbial host cell, and can be optimized to maximize production or melatonin or another desired compound by varying culture conditions and media components as is well-known in the art.

For a recombinant *Escherichia coli* cell, exemplary media include LB medium and M9 medium (Miller, Journal of Experiments in Molecular Genetics, 431-433, Cold Spring Harbor Laboratory, New York, 1972), optionally supplemented with one or more amino acids. When an inducible promoter is used, the inductor can also be added to the medium. Examples include the lac promoter, which can be activated by adding isopropyl-beta-thiogalactopyranoside (IPTG) and the GAL/BAD promoter, in which case galactose/arabinose can be added. The culturing can be carried out a temperature of about 10 to 40 °C for about 3 to 72 hours, if desired, with aeration or stirring.

For a recombinant *Bacillus* cell, culturing can be carried out in a known medium at about 30 to 40 °C for about 6 to 40 hours, if desired with aeration and stirring. With regard to the medium, known ones may be used. For example, pre-culture can be carried out in an LB medium and then the main culture using an NU medium.

For a recombinant yeast cell, Burkholder minimum medium (Bostian, K. L., et al. Proc. Natl. Acad. Sci. USA, volume 77, 4505 (1980)), SD medium containing 0.5% of Casamino acid (Bitter, G. A., et al., Proc. Natl. Acad. Sci. USA, volume 81, 5330 (1984), and Delft medium (Verduyn et al., Yeast 1992, 8, 501-517) can be used. The pH is preferably adjusted to about 5-8. For example, a synthetic medium may contain, per litre: (NH4)2SO4, 5 g; KH2PO4, 3 g; MgSO4.7H2O, 0.5 g; EDTA, 15 mg; ZnSO4.7H2O, 4.5 mg; CoCl2.6H2O, 0.3 mg; MnCl2.4H20, 1 mg; CuSO4 5H2O, 0.3 mg; CaCl2.2H2O, 4.5 mg; FeSO4.7H2O, 3 mg; NaMoO4.2H2O, 0.4 mg; H3BO3, 1 mg- KI, 0.1 mg; and 0.025 ml silicone antifoam(BDH). Filter-sterilized vitamins can be added after heat sterilization (120°C), to final concentrations per litre of: biotin, 0.05 mg; calcium pantothenate, 1 mg; nicotinic acid, 1 mg; inositol, 25 mg; thiamine HCl, 1 mg; pyridoxine HCl, 1 mg; and para- aminobenzoic acid, 0.2 mg. The medium can then be adjusted to pH6 with KOH. Culturing is preferably carried out at about 20 to about 40 °C, for about 24 to 84 hours, if desired with aeration or stirring.

In one embodiment, no L-tryptophan is added to the medium. In another embodiment, no L-tryptophan or THB is added to the medium, so that the production of melatonin or its precursors or related compounds rely on endogenously biosynthesized substrates.

Using the method for producing 5HTP, L-DOPA, tyrosine, m-tyrosine, hydroxytyrosol, melatonin, serotonin or N-acetyl-serotonin according to the invention, a yield of at least about 0.5%, such as at least about 1%, such as at least 5%, such as at least 10%, such as at least 20%, at least 30%, at least 40%, at least 50%, at least 60%, at least 70%, at least 80% or at least 90% of the theoretically possible yield can be obtained from a suitable carbon source, such as glucose.

Isolation of 5HTP, L-DOPA, tyrosine, m-tyrosine, hydroxytyrosol, melatonin, N-acetylserotonin or serotonin from the cell culture can be achieved, e.g., by separating the compound from the cells using a membrane, using, for example, centrifugation or filtration methods. The product-containing supernatant is then collected. Further purification of the desired compound can then be carried out using known methods, such as, e.g., salting out and solvent precipitation; molecular-weight-based separation methods such as dialysis, ultrafiltration, and gel filtration; charge-based separation methods such as ion-exchange chromatography; and methods based on differences in hydrophobicity, such as reversed-phase HPLC; and the like. In one embodiment, ion-exchange chromatography is used for purification of serotonin. An exemplary method for serotonin purification using cation-exchange chromatography is described in Chilcote (1974) (Clin Chem 20(4):421-423). In one embodiment, reverse-phase chromatography is used for separation and/or purification of serotonin, N-acetylserotonin, or melatonin. An exemplary method for purification of these indolamines using reversed-phase chromatography is described in Harumi et al., (1996) (J Chromatogr B 675:152-156).

Once a sufficiently pure preparation has been achieved, suitable excipients, stabilizers can optionally be added and the resulting preparation incorporated in a composition for use in preparing a product such as, *e.g.,* a dietary supplement, a pharmaceutical, a cosmeceutical, or a nutraceutical. For a dietary supplement comprising melatonin, each serving can contain, *e.g.,* from about 0.01 mg to about 100 mg melatonin, such as from about 0.1 mg to about 10 mg, or about 1-5 mg, such as 2-3 mg. Emulsifiers may be added for stability of the final product. Examples of suitable emulsifiers include, but are not limited to, lecithin (e.g., from egg or soy), and/or mono- and di-glycerides. Other emulsifiers are readily apparent to the skilled artisan and selection of suitable emulsifier(s) will depend, in part, upon the formulation and final product. Preservatives may also be added to the nutritional supplement to extend product shelf life. Preferably, preservatives such as potassium sorbate, sodium sorbate, potassium benzoate, sodium benzoate or calcium disodium EDTA are used.

### EXAMPLE 1

### Media and growth conditions

All strains were maintained at 37°C in LB (Lennox) Broth (Sigma-Aldrich), 2xYT or M9 minimum media containing 1xM9 minimal salts (BD Difco^{™}), 2 mM MgSO₄, 100 µM CaCl₂, 500-fold diluted trace minerals (10 g/l FeCl₃·6H₂O, 2 g/l ZnSO₄·7H₂O, 0.4 g/l CuCl₂·2H₂O, 1 g/l MnSO₄·H₂O, 0.6 g/l CoCl₂·6H₂O, and 1.6 mM EDTA, pH 8.0), 1x ATCC^{®} Vitamin Supplement (ATCC MD-VS^{™}), and 0.2% glucose (w/v). Unless stated otherwise, required supplementations were carried out as following: L-phenylalanine at 50 mg/l, L-tyrosine at 30 mg/l, folates at 10 mg/l, chloramphenicol at 25 mg/l, kanamycin at 25 mg/l, and spectinomycin at 50 mg/l.

### Plasmids

The pPheH plasmid carries a codon-optimized *pheH* from C. *violaceum.* The pTHBsc plasmid carries the *pcd, qDPR, pts* and *spr* genes. The *pcd and qDPR* genes are from C. *violaceum* while the *pts* and *spr* genes are from *Rattus norvegicus.* The pHM1 plasmid was derived from pTHBscEv, an evolved pTHBsc plasmid isolated after PheH-dependent laboratory evolution, containing the human TpH2 gene and a sacB gene, which causes plasmid instability upon exposure to sucrose. All synthetic genes were optimized for *E. coli* expression.

### Metabolite analysis by LC-MS

LC-MS data was collected on OrbiTrap Fusion High Resolution Mass Spectrometer system coupled with an Ultimate 3000 UHPLC pump (Thermo, San Jose Ca). Samples were held in the autosampler at a temperature of 10.0°C during the analysis. 1µL Injections of the sample were made onto a Thermo HyperSil Gold PFP HPLC column, with a 3 um particle size, 2.1 mm i.d. and 150 mm long. The column was held at a temperature of 35.0°C. The solvent system used was Solvent A "Water with 0.1% formic acid" and Solvent B "Acetonitrile with 0.1% formic ". The Flow Rate was 1.000 ml/min with an Initial Solvent composition of %A = 95, %B = 5 held until 0.50 min, the solvent composition was then changed following a Linear Gradient until it reached %A = 70.0 and %B = 30.0 at 1.50 min. The solvent composition was then changed following a Linear Gradient until it reached %A = 5.0 and %B = 95.0 at 2.00 min This was held until 2.50 min when the solvent was returned to the initial conditions and the column was re-equilibrated until 3.00 min. The first 0.25 min of the run was diverted to waste using the divert valve, following which the column eluent flowed directly into the Heated ESI probe of the MS which was held at 325°C and a voltage of 3500 V. Data was collected in positive ion mode over the mass range 50 to 1000 m/z at a resolution of 15.000. The other MS settings were as follows, Sheath Gas Flow Rate of 60 units, Cone Gas Flow Rate of 20 units Cone Temp was 275°C.

### H₄-BPt optimization by Phenylalanine-hydroxylase dependent laboratory evolution

Patent applications PCT/EP2013/054019 and PCT/EP2014/068967 described a method for the production of 5-hydroxytryptophan and melatonin by heterologous expression of human tryptophan hydroxylase (TpH) in *E.coli.* In addition to the desired biosynthetic genes, teterhydrobiopterine (H₄-BPt) synthesis and recycling pathways consisting of four genes have to be introduced to accommodate TpH cofactor usage. Preliminary studies indicated that the rate-limiting step was human TpH turnover, possibly due to limitations in cofactor supply.

To overcome this challenge, a phenylalanine-hydroxylase dependent growth-coupled selection was designed. The key to this design was: 1) to construct an *E*. *coli* tyrosine auxotroph; 2) to introduce an H₄-BPt-dependent phenylalanine hydroxylase (PheH) that converts phenylalanine to tyrosine. The chosen PheH was from *Chromobacterium violaceum* (SEQ ID NO:58) and the chosen background strain was HL1308. The *E*. *coli* HL1308 strain was constructed from JW2300 of the Keio collection (Baba et al, 2006) with *tyrA, recA* and *galE* deletions further introduced according to Datsenko and Wanner (2000) or by P1 transduction. The HL1305 strain is genotypically similar to HL1308 except the *tyrA* deletion. Deletion of *tyrA,* donated by Δ*tyrA,* resulted in a tyrosine auxotroph while Δ*foIX* was necessary to inactivate *de novo* biosynthesis of tetrahydromonapterin known to be an electron donor of PheH from *Pseudomonas aeruginosa* (Pribat et al, 2010). The recA and *galE* genes were removed respectively to minimize DNA recombination during laboratory evolution and to acquire a high DNA uptake efficiency. Constitutive expression and regulations of the heterologous genes were enabled via a hybrid sequence of the native TyrR-repressible *aroF* promoter sequence (Cobbett, 1988) and the translation leader sequence of the *rpsA* gene (Tchufistova et al, 2003).

Preliminary results confirmed HL1308 strain was unable to grow without tyrosine supplementation. On the other hand, its tyrosine auxotrophy was reverted upon transformation of the pTHBsc and pPheH plasmids. The transformed strain grew at an approximately half the rate of the control strain (HL1305) with phenylalanine supplementation; however, there was no observable growth in the absence of phenylalanine.

Subsequently, the transformed strain was subjected to 280 generations of manual passage using shake flasks. The procedure was to inoculate 25 ml fresh phenylalanine supplemented M9 in a 250 ml flask to an initial density which was expected to reach an OD₆₀₀ of 0.2-0.4 after 24 hour of incubation in an orbital 311DS shaker (Labnet) at 37°C and 250 rpm. This serial transfer was repeated for 16 days. The initial phenylalanine concentration was 50 mg/l and was lowered to 5 mg/l starting from day 7. Colonies were isolated at Day 6 and Day 16 and their growth rates were determined.

Adapted isolates from the high phenylalanine conditions displayed a wide range of growth response from barely growth to a growth rate comparable to HL1305 in the presence of 50 mg/l of phenylalanine. This result was in contrast to the ones recovered from the low phenylalanine environment whereas all strains exhibited significant improvement in growth with a median rate of 0.5±0.03 h⁻¹ and all were able to propagate in M9 without phenylalanine supplementation. The wider distribution in the growth rates of high-phenylalanine adapted isolates led us to hypothesize that certain strains in the population were evolved to become a tyrosine secretor when an excess amount phenylalanine was present. We examined the hypothesis by feeding 0.5 g/l of phenylalanine to the best-grown strain, THB402F3C9, and confirmed ~0.1 g/l of tyrosine was secreted after overnight growth.

Plasmids originated from adapted isolates were sequenced. It appeared the pTHBsc plasmid from all isolates lacked the H₄-BPt biosynthesis and recycling genes except *pcd* thus all were H₄-BPt null strains. In addition, there were neither mutations presented in the *pheH* and *pcd* genes nor on the non-translational regions of the plasmids. Combining both evidences, it was reasoned *E. coli* cells must have repurposed its own metabolism to fulfil PheH cofactor requirement during adaptation and pterine-4a-carbinolamine dehydratase encoded by *pcd* was the only additional requirement.

A series of deletion mutants were made to elucidate possible native PheH cofactor in *E. coli.* Since folic acids and 6-carboxy-tetrahydropterin (H₄-CPt) share the same metabolic precursor as of H₄-BPt, it was reasoned one of them or intermediates were activated as the electron donor of PheH. To test this idea, their respective biosynthesis genes, *queD* and *nudB,* were deleted from the HL1308 Δ*tyrR* strain (HL1310). The Δ*queD* strain (HL1312) exhibited no obvious growth defect upon transformation of pTHBscEv and pPheH (Table 2). On the other hand, the Δ*nudB* strain (HL1314) exhibited severely impaired PheH-dependent growth while restoration of the tyrosine auxotrophy in HL1314 led to a strain (HL1314R) whose growth rate was comparable to control (Table 2).

Physiological characterizations indicated folic acids and their intermediates must have contributed to the turnover of PheH in the evolved strains. The 6-hydroxymethyl-dihydropterin (6-CH₂OH-H₂Pt) is a metabolic intermediate of folic acids biosynthesis. Enzymatic characterizations have shown FolM reduces 6-CH₂OH-H₂Pt to 6-hydroxymethyl-tetrahydropterin (6-CH₂OH-H₄Pt) *in vitro* (Pribat et al, 2010) (**Figure 1**)**.** Due to its structural resemblance to H₄-BPt (**Figure 1** insert), a Δ*foIM* strain was made but growth measurements showed mutant behaved similar to HL1310 eliminating 6-CH₂OH-H₄Pt as a functional complement (**Table 2**)**.** This result was supported by the making of HL1330, a *ΔpabA* strain that is capable to synthesize all intermediates of the folic acids biosynthesis pathway from dihydroneopterin triphosphate (H₂-NPtP₃) to dihydropteroate (H₂-Pte) except folic acids (**Figure 1**)**.** Growth results showed folates supplementation could rescue JW3323 (BW25113 Δ*pabA*) (Baba et al, 2006) in M9 but not PheH-dependent growth of HL1330. These results have led us to conclude the tetrahydrofolic acids were the acting donor of PheH in the evolved strain.

**Table 2: Growth measurements of various mutants ^{T}**

| **Strains** | **Genotype** | **growth rate, h⁻¹** | **inoculation OD₆₀₀** | **OD₆₀₀ after 8 h** | **comments** |
|---|---|---|---|---|---|
| HL1305 | BW25113 Δ*folX* | 0.59 | 0.035 | 0.593 | control strain |
| | *ArecA* Δ*galE* | | | | |
| HL1308¹ | BW25113 Δ*folX* | 0.244 | 0.025 | 0.178 | |
| | Δ*tyrA* Δ*recA ΔgalE* | | | | |
| HL1310¹ | HL1308 Δ*tyrR* | 0.33 | 0.023 | 0.382 | |
| HL1312¹ | HL1310 Δ*queD* | 0.27 | 0.024 | 0.155 | |
| HL1314¹ | HL1310 Δ*nudB* | *no growth* | 0.02 | 0.051 | |
| HL1314R | HL1314 *tyrA*⁺ | 0.58 | 0.02 | 0.6 | |
| HL1326¹ | HL1310 Δ*folM* | 0.3 | 0.027 | 0.289 | |
| JW3323 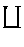 | BW25113 Δ*pabA* | 0.4 | 0.036 | 0.488 | Keio collection |
| HL1330^{1,} 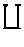 | HL1310 Δ*pabA* | *no growth* | 0.022 | 0.035 | |
| THB402F3C9 | HL1308 Δ*rpsA*² | 0.58 | 0.027 | 0.61 | evolved strain |
| | *rpoB*:E546K | | | | |
| | rpoC:I1357S | | | | |
| ^{T}: Growth measurements were performed in M9 minimum media with 50 mg/l phenylalanine. | | | | | |
| 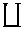 : Folic acids at 10 mg/l were added. | | | | | |
| ¹: Cells were transformed with pTHBscEv and pPheH. | | | | | |
| ²: The *rpsA* gene is inactivated by a frameshift deletion at the 9th nucleotide after the ATG start codon. | | | | | |

### Tryptophan-hydroxylase dependent laboratory evolution

Based on the phenylalanine-hydroxylase study, we hypothesized that it was possible to use native *E. coli* compounds (*e.g.* folate species) to support AAH activity with functional expression of the *pcd* gene being the only requirement. Since it was known that TpH is capable of phenylalanine turnover to form tyrosine *in vitro,* thus it is plausible to adjust TpH by evolution using the same principle.

In the subsequent evolution, TpH replaced the PheH in the previous evolution. This was done by inserting a Ptrc (Trc promoter)-regulated human TpH gene (encoding SEQ ID NO: 13) onto pTHBscEv along with a counter-selectable sacB gene. The resulting plasmid, pHM1, was introduced to a tyrosine auxotroph HM30 strain, which was derived from JW2581 of the KEIO collection but with the kanamycine-resistant marker at the *tyrA* locus removed. The final transformed strain was referred as HM35. The HM35 cells were then growth-adapted for high growth rate in the presence of phenylalanine.

At the end of experiment, 94 isolates were picked and were grown in M9 medium supplemented with 200 mg/l of tryptophan and 76 mg/l of tyrosine. The amount of 5HTP formed in the exo-metabolom was measured, it was observed some isolates were able to accumulate more than 10 mg/l of 5HTP and this was ~10-fold more than their parent strain (HM35).

A total of 7 high 5HTP-producers were subjected to total DNA sequence analysis. Three mutations were found common in all strains: FolE (T198I), YnbB (V197A) and TpH (E2K, in SEQ ID NO: 13). Both FolE (T198I) and YnbB (V197A) mutations were genomic changes while the TpH (E2K) mutation occured on the pHM1 plasmid. Further analyses indicated that the YnbB (V197A) mutation might not be any functional importance since it was also found in the parent HM35 strain. However, without being limited to theory, the T198I change in FolE may have resulted in increased supply of a TpH cofactor as FolE encoding for GTP cyclohydrolase I is the first enzyme leading to tetrahydrofolates, tetrahydromonapterin and others which may act as TpH cofactors in *E. coli.* This is a particularly interesting result, since preliminary studies have indicated that the catalytic effectiveness of FolE cannot be easily increased by gene overexpression. Furthermore, a proteomic study revealed that the E2K mutation resulted in ~20 fold increase in TpH abundance in contrast to the wild-type form.

### EXAMPLE 2

### Directed TpH Engineering

It was found that *Homo sapiens* TpH2, *i.e*., the fragment set forth as SEQ ID NO: 13; hsTpH2, was sensitive to p-chlorophenylalanine. However, mutations at residues N97 and/or P99 were found to confer resistance to p-chlorophenylalanine and to exhibit improved 5HTP biosynthesis after growing cells in the presence of 100 mg/l of tryptophan overnight at 37°C. A further, saturated mutagenesis, study found that isoleucine (I) was a beneficial amino acid change at residue N97, while cysteine (C), aspartic acid (D), leucine (L) and glutamine (Q) were shown to be beneficial at residue P99. In particular, the combined changes N97I/P99D in hsTpH2 showed a >15% increase in 5HTP production in the presence of 100 mg/l tryptophan and the combined changes N97I/P99C in hsTpH2 showed a >25% increase in 5HTP biosynthesis, over the parent TPH2 sequence (SEQ ID NO: 13) after acquiring the E2K mutation.

### EXAMPLE 3

### Effectiveness of newly evolved TpH background strain using Schistosoma mansoni TpH

One of the 7 evolved high 5HTP-producers was selected to further evaluate if the mutations identified were only specifically beneficial to hsTpH2 or could be widely applicable to others. The chosen evolved strain was first cured to lose the evolution plasmid (e.g. the hsTpH gene) and this was immediately followed by re-introducing the *E.coli* tyrA gene. Upon restoration of the strain's tyrosine auxotrophy, the resulting strain was transformed with pHM2, which is identical to pHM1 used in the earlier evolution study except that the hsTpH gene was replaced with a *Schistosoma mansoni* TpH gene (SEQ ID NO:9). The 5HTP production of the resulting strain was compared to a wild-type strain carrying pHM2 in the presence of 100 mg/l tryptophan. Results showed the wild-type transformants could only produce ~0.05 mg/l 5HTP while the newly evolved background strain transformants accumulated >20 mg/l. These production results demonstrated that the mutations acquired in the evolved background strain were not only beneficial to hsTpH but also to other TpHs; possibly applicable also to other aromatic amino acid hydroxylases (*e.g.* tyrosine hydroxylase).

### EXAMPLE 4

### Effect of FolE mutation

This Example shows that the FoIE(T198I) mutation is a beneficial mutation towards TpH functionality.

It was assumed the FolE mutation was the main contributor on improving TpH turnover. To challenge this assumption, the FoIE(T198I) mutation was introduced into the wild-type BW25113 strain by site-directed mutagenesis using CRISPR. All strains were transformed with a plasmid carrying the human TPH and *C. violaceum* PCD genes. The transformed cells were grown in M9 medium containing 100 mg/l tryptophan overnight and extracellular metabolites were subjected to analytical measurements. The results are summarized in Table 3.

**Table 3: Importance of FoIE(T198I) on in vivo hsTpH¹ turnover in the presence of 100 mg/l of tryptophan**

| Genotype | 5HTP (mg/l) | Indole (mg/l) | 5-hydroxyindole (mg/l) |
|---|---|---|---|
| wild-type | n.d. | 85±5 | n.d. |
| ΔTnaA | 40±2 | n.d. | n.d. |
| FolE(T198I) | n.d. | n.d. | n.d. |
| ΔTnaA FolE(T198I) | 129±30 | n.d. | n.d. |
| ¹: hsTpH carries E2K, N97I and P99C mutations in SEQ ID NO:13 | | | |
| n.d.: not detected | | | |

As shown in Table 3, wild-type hsTpH expressing cells did not produce 5HTP under tryptophan feeding conditions. However, 5HTP production could be observed with TnaA deletion, which prevented tryptophan and 5HTP degradation. Further 3-folder enhancement on 5HTP production was achieved with FolE(T198I) on ΔTnaA background. Accordingly, the FolE change is a beneficial mutation towards TpH functionality.

### EXAMPLE 5

### Tyrosine hydroxylase activity

This Example shows that the FolE(T198I) mutation is a beneficial mutation also for supporting tyrosine hydroxylase functionality.

Since both tyrosine hydroxylase and tryptophan hydroxylase belong to the same class of enzyme (i.e., utilizing the same cofactor for oxidation), it was also expected FolE(T198I) would benefit tyrosine hydroxylase activity in terms of converting L-tyrosine to L-3,4-dihydroxyphenylalaine (L-DOPA). Both wild type *E. coli* and FolE(198I) cells were transformed with a plasmid carrying either the wild-type or truncated rat TH gene expressed from a trc promoter. Additionaly, the proline residue of the 2^{nd} amino acid of the truncated gene was replaced with lysine. The transformed cells were grew in M9 containing 100 mg/l tyrosine overnight and extracellular metabolites were subjected to analytical measurements. As summarized in Table 4, it was shown that the presence of FolE mutation enhanced L-DOPA formation by about 3-fold when compared to the ones lacking the mutation. Furthermore, it was also shown that removal of the N-terminal mammalian signalling amino acids and substituting the 2^{nd} amino acid from proline to lysine additionally enhanced L-DOPA formation about 4-fold. It was therefore a total of c.a. 10-fold improvement achieved by combining all the modifications. Without being limited to theory, it is believed that the FolE mutation could benefit other enzymes of the same class, including phenylalanine hydroxylase (EC 1.14.16.1), phenylalanine 3-hydroxylase (EC 1.14.16.7), tyrosine 3-hydroxylase (EC 1.14.16.2), anthranilate 3-monooxygenase (EC 1.14.16.3), mandelate 4-monooxygenase (EC 1.14.16.6) and Alkylglycerol monooxygenase (EC 1.14.16.5).

**Table 4: Importance of FoIE(T198I) on in vivo tyrosine hydroxylase turnover in the presence of 100 mg/l of tyrosine**

| Tyrosine hydroxylase¹ | FolE(T198I) | L-3,4-dihydroxyphenylalanine (LDOPA) (mg/g dry cell weight) |
|---|---|---|
| wild-type² | No | 2.88±0.1 |
| wild-type² | Yes | 7.25±0.69 |
| P2K Δ(3-157) | No | 10.62±0.71 |
| P2K Δ(3-157) | Yes | 30.3±1.43 |
| ¹: tyrosine hydroxylase from *Rattus norvegicus* | | |
| ²: NCBI accession number: NP_036872.1 | | |

### EXAMPLE 6

### Comparing FolE overexpression with FolE mutation

This Example shows that overexpression of the wild-type *folE* gene did not achieve an FoIE(T198I) effect.

Gene overexpression has often been used to enhance *in vivo* enzymatic turnover. As deterimined in Example 1, TPH-dependent cell growth basing on phenylalanine to tyrosine conversion was an effective way to reflect TPH turnover and its associated networked genes, including its cofactor supply. Therefore, a TPH-dependent growth experiment was set up to compare the effectiveness of the FolE mutation and gene overexpression. Overexpression of the *folE* gene was achieved by introducing a *folE* bearing plasmid with its native *E. coli* promoter intact. The TPH-dependent HM30 cells were transformed with two plasmids: a TPH/PCD bearing plasmid and the concerning *folE* bearing plasmid. The transformed strains were grown in M9 in the presence of 100 mg/l phenylalanine at 37°C along with control strains carrying either wild-type chromosomal FolE or FoIE(T198I) in additional to TPH/PCD expression from a plasmid. Cell growth was measured at regular intervals.

The results are summarized in Figure 2. As shown, strains carrying a chromosomal FolE mutation (gFolE*) exhibited rapid TPH-dependent growth during the time course of this experiment. This was in contrast to the native chromosomal variant (gFolE), which barely grew. Although it was interesting to see cells with overexpression of wild-type FolE (gFolE+pFolE) exhibited elevated cell growth yet its growth was not as rapid as the single copy chromosomal mutant. Furthermore, it was observed that cells carrying a hybrid of native and mutated FolE genes (gFolE+pFolE*) showed improved growth; however, its growth was not as good as homogeneous chromosomal mutant (gFolE*). The reason was believed to be that since the T198 residue was located in a peptide segment affecting FolE oligomerization, a hybrid pool of the FolE proteins would ultimately affect the polymerization of the FolE complex and hence its overall activity.

### EXAMPLE 7

### Screen of FolE library

This Example shows that the conformation of the FolE oligomerization helix (Ser207-Arg219) affects GTP cyclohydrolase I activity.

Structural analysis showed that the T198 residue is located in the 4^{th} β-strand that goes in a close proximity to a α-helix defined by Ser207 to Asn222, which can be involved in oligomerization of the FolE complex. Accordingly, T198 may play a role in the FolE oligomerization. Specifically, its mutation would affect the hydrophobic interaction with Leu215 and subsequently the positioning of Ser207-Arg219 helix and overall polymerization of the FolE complex. It can therefore be predicted that additional beneficial FolE mutations can be located in the segments corresponding to residues D97-E112, residues K121-D130, residues N170-H180, residues S193-L200 and residues S207-N222.

A *folE* gene random library was constructed and was screened by growth according to Example 6. None-redundant growth-improved mutants and their corresponding mutations are summarized in Table 5.

**Table 5: Summary of beneficial FolE mutations identified by random mutagenesis**

| Mutants | FolE mutations | Mutant growth rate, h⁻¹ | Improvement over wt |
|---|---|---|---|
| HMP1058¹ | Wild type | 0.19 | (1) |
| HMP1059² | T198I | 0.25 | 1.32 |
| P1-01 | I67V, T117I, A125D, H221R | 0.32 | 1.68 |
| P1-02 | V179A | 0.32 | 1.68 |
| P1-03 | M99I | 0.36 | 1.89 |
| P1-04 | E62K, N170K, L215P | 0.41 | 2.16 |
| P1-05 | V102M, L215P | 0.35 | 1.84 |
| P1-06 | T198S | 0.32 | 1.68 |
| P1-07 | Q157L, H212R | 0.36 | 1.89 |
| P1-08 | V28L, L215P, N222I | 0.4 | 2.11 |
| P1-10 | F214S | 0.32 | 1.68 |
| P1-11 | T108N, I133F, E213K | 0.35 | 1.84 |
| P1-12 | Q157L, H212R | 0.37 | 1.95 |
| P2-01 | S5C, D57V, L215Q | 0.36 | 1.89 |
| P2-02 | H29Y, I75V, V179M | 0.32 | 1.68 |
| P2-03 | A14V, E46D, M61I, D97V | 0.36 | 1.89 |
| P2-04 | V28A, G42D, E213K | 0.4 | 2.11 |
| P2-05 | N52K, A68S, S207R | 0.36 | 1.89 |
| P2-06 | L200P | 0.36 | 1.89 |
| P2-07 | A41G, K129N, I133F | 0.3 | 1.58 |
| P2-09 | S3L, K184R, S199Y | 0.32 | 1.68 |
| P2-11 | H12R, N170D, G187S | 0.39 | 2.05 |
| ¹: In Example 6 illustrated as a mutant of gFolE+pFolE | | | |
| ²: In Example 6 illustrated as a mutant of gFolE+pFolE* | | | |
| .: cell growth was measured in M9 with 100 mg/l of phenylalanine at 37°C overnight. | | | |

As shown in Table 5, mutants carrying E213K and L215P or L215Q showed the most significant growth improvement following by D97V and M99I of the D97-E112 segment, N170K and N170D of the N170-H180 segment, and L200P, S207R and H212R of the S207-N222 segment. Other mutations included V102M of the D97-E112 segment, A125D and K129N of the K121-D130 segment, V179A and V179M of the N170-H180 segment, T198S and S199Y of S193-L200 segment, and F214S, H221R of the S207-N222 segment. Overall, these results strongly suggest that mutations altering FolE oligomerization can significantly alter the overall turnover rate of GTP cyclohydrolase I (FolE).

### LIST OF REFERENCES

Lin et al., ACS synthetic biology 2014;3:497-505
Ehrenworth et al., ACS Synth Biol. 2015 Dec 18;4(12):1295-307.
Yamamoto et al., Metab Eng 2003;5:246-25
Hara and Kino, AMB Express 2013;3:70
McKenzie G and Nancy LC (2006) BMC Microbiol 6:39
Nar et al., PNAS USA 1995;92:12120-5
Rebelo et al., J. Mol. Biol. 2003;326:503-516
Pribat et al., J Bacteriol 2010a; 192(2):475-82
Pribat et al., Plant Cell 2010b;22(10):3410-22
Baba et al., Mol Syst Biol 2006:2: 1-11
Cobbett et al., Mol Microbiol 1988;2: 377-383
Datsenko and Wanner Proc Natl Acad Sci USA 2000;97: 6640-6645
Pribat et al., J Bacteriol 2010:192: 475-482
Satoh et al., Metabolic engineering 2012;14:603-610
Tchufistova et al., Nucleic Acids Res 2003:31: 6996-7002
WO 2013/127914 A1, WO 2013/127915 A1 and WO 2015/032911 A1 (Danmarks Tekniske Universitet)
US 2014/134689 AA (University of California)
US Patent No. 7,807,421 (Asubio Pharma Co., Ltd)

## Claims

1. A variant of *E. coli* GTP cyclohydrolase I (GCH1) comprising one or more mutations, wherein,
in an *E. coli* cell comprising a pterin-4a-carbinolamine dehydratase (PCD) and at least one of a tryptophan hydroxylase (TPH), a tyrosine hydroxylase (TH) and a phenylalanine hydroxylase (PheH), the variant provides for an increased hydroxylation activity of at least one of the TPH, TH and PheH as compared to native *E. coli* GCH1,
wherein
(i) the variant has at least 94% sequence identity to native *E. coli* GCH1 having the sequence of SEQ ID NO:16 and at least one of the one or more mutations is in an amino acid residue selected from the group consisting of D97, M99, T101, V102, A125, K129, N170, V179, T196, S199, L200, S207, H212, E213, F214, L215 and H221,
wherein the mutation in N170 is N170K, N170D or N170L; the mutation in V179 is V179A; the mutation in H212 is H212R or H212K; and the mutation in H221 is H221R or H221K; or
(ii) the variant has at least 98% sequence identity to native *E. coli* GCH1 having the sequence of SEQ ID NO:16 and at least one of the one or more mutations is in the amino acid residue T198, wherein the mutation in T198 is not T198P.

2. The variant of claim 1, wherein the variant
(a) comprises a mutation selected from D97V, D97L, D97A, D97T, M99C, M99T, M99V, M99L, M99I, T101I, T101V, T101L, V102M, N170K, N170D, N170L, V179A, V179M, T196I, T196V, T196L, S199Y, S199F, L200P, L200C, L200S, L200A, S207R, S207K, S207M, H212R, H212K, E213K, E213R, F214A, F214G, F214S, L215P, L215Q, L215N, L215D, L215T, L215S, L215G, L215A, L215C, L215F, L215M, H221R and H221K, or a combination thereof;
(b) has at least 98% or at least 99% sequence identity to the native *E. coli* GCH1;
(c) provides for a hydroxylation activity of at least about 120% as compared to native *E. coli* GCH1; or
(d) a combination of any two or more of (a) to (c).

3. The variant of claim 1, comprising amino acid substitutions selected from
(a) I67V, T117I, A125D, H221R;
(b) V179A;
(c) M99I;
(d) E62K, N170K, L215P;
(e) V102M, L215P;
(f) Q157L, H212R;
(g) V28L, L215P, N222I;
(h) F214S;
(i) T108N, I133F, E213K;
(j) S5C, D57V, L215Q;
(k) H29Y, I75V, V179M;
(l) A14V, E46D, M61I, D97V;
(m)V28A, G42D, E213K;
(n) N52K, A68S, S207R;
(o) L200P;
(p) A41G, K129N, I133F;
(q) S3L, K184R, S199Y; and
(r) H12R, N170D, G187S.

4. The variant of any one of the preceding claims, comprising a mutation selected from F214S, V179A, M99I and L200P.

5. The variant of claim 1, wherein the variant
(a) comprises a mutation selected from T198I, T198V, T198S and T198L;
(b) has at least 99% sequence identity to the native *E. coli* GCH1;
(c) provides for a hydroxylation activity of at least about 120% as compared to native *E. coli* GCH1; or
(d) a combination of any two or more of (a) to (c).

6. A variant of a tryptohan hydroxylase (TPH), comprising
a segment corresponding to residues E147 to T460 of *Homo sapiens* TPH having the sequence of SEQ ID NO:3,
an N-terminal methionine residue, and
a mutation in each residue corresponding to residues E147, N242 and P244 in SEQ ID NO:3,
wherein the variant is a variant of a *Homo sapiens* (SEQ ID NO:3), *Schistosoma mansoni* (SEQ ID NO:9), *Gallus gallus* (SEQ ID NO:6), *Sus scrofa* (SEQ ID NO:5), *Equus caballus* (SEQ ID NO:8), *Bos taurus* (SEQ ID NO:4), *Mus musculus* (SEQ ID NO:7) or *Oryctolagus cuniculus* (SEQ ID NO:1):TPH, and wherein the segment in the variant has at least about 80% sequence identity to the segment in the native sequence,
wherein the variant provides for a higher tryptophan hydroxylation activity than the native TPH, and
wherein
(a) the mutation in the residue corresponding to residue E147 is an amino acid substitution selected from 147K, 147R and 147H;
(b) the mutation in the residue corresponding to residue N242 is the amino acid substitution 242I; and
(c) the mutation in the residue corresponding to P244 is selected from 244C, 244D, 244L and 244Q.

7. The variant of claim 6, comprising or consisting of the amino acid sequence of SEQ ID NO: 13 with mutations E2K, N97I and P99C.

8. A nucleic acid sequence encoding
(a) the variant of *E. coli* GCH1 of any one of claims 1 to 5;
(b) the variant *Homo sapiens* TPH of any one of claims 6 to 7; or
(c) both of (a) and (b).

9. A recombinant microbial cell comprising the variant *E. coli* GCH1 of any one of claims 1 to 5 or the nucleic acid sequence of claim 8(a).

10. The recombinant microbial cell of claim 9, further comprising nucleic acid sequences encoding a PCD and a monooxygenase, optionally wherein each nucleic acid sequence is operably linked to an inducible, a regulated or a constitutive promoter, and/or wherein at least the nucleic acid sequence encoding the variant of *E. coli* GCH1 is chromosomally integrated.

11. The recombinant microbial cell of claim 10, wherein
(a) the PCD is selected from *Chromobacterium violecum, Homo sapiens, Pseudomonas aeruginosa* and *Rattus norvegicus;* or a functionally active variant, homolog or fragment thereof;
(b) the monooxygenase is a TPH selected from a *Schistosoma mansoni, Homo sapiens, Gallus gallus, Bos taurus, Sus scrofa, Equus caballus, Mus musculus* and *Oryctolagus cuniculus* TPH; or a functionally active variant, homolog or fragment of any thereof, optionally the variant TPH of any one of claims 6 and 7;
(c) the monooxygenase is a TH selected from *Rattus norwegicus, Homo sapiens, Mus musculus, Bos taurus, Gallus gallus* or a functionally active variant, homolog or fragment thereof;
(d) the monooxygenase is a PheH selected from *Chromobacterium violaceum, Xanthomonas campestris pv. Viticola, Pseudomonas aeruginosa, Pseudomonas putida, Homo sapiens, Mus musculus, Streptomyces coeruleorubidus* or a functionally active variant, homolog or fragment thereof; or
(e) a combination of (a) and (b), (a) and (c) or (a) and (d).

12. The recombinant microbial cell of any one of claims 9 to 11, which is derived from a bacterial cell, a yeast cell, a filamentous fungal cell, or an algal cell.

13. The recombinant microbial cell of any one of claims 9 to 12, which is derived from an *Escherichia,* a *Saccharomyces,* a *Schizosaccharomyces,* a *Corynebacterium,* a *Bacillus* or a *Streptomyces* cell, such as an *E. coli* cell.

14. The recombinant microbial cell of any one of claims 9 to 13, further comprising
(a) a nucleic acid sequence encoding a 5HTP decarboxylase (ADDC);
(b) nucleic acid sequences encoding an ADDC and a serotonin acetyltransferase (AANAT);
(c) nucleic acid sequences encoding an ADDC, an AANAT, and an acetylserotonin O-methyltransferase (ASMT); or
(d) nucleic acid sequences encoding a dopa decarboxylase, a tyramine oxidase and an alcohol dehydrogenase.

15. A method of producing one or more oxidation products of an aromatic amino acid, comprising culturing the recombinant microbial cell of any one of claims 9 to 14 in a medium comprising a carbon source, and, optionally, isolating the oxidation product.

16. The method of claim 15, wherein the oxidation product comprises at least one of 5HTP, L-DOPA, tyrosine, m-tyrosine, hydroxytyrosol, serotonin and melatonin.

## Patentansprüche

1. Variante von *E.-coli*-GTP-Cyclohydrolase I (GCH1), die eine oder mehrere Mutationen umfasst, wobei
in einer E-coli-Zelle, umfassend eine Pterin-4o-Carbinolamin-Dehydratase (PCD) und mindestens eine aus einer Tryptophan-Hydroxylase (TPH), einer Tyrosin-Hydroxylase (TH) und einer Phenylalanin-Hydroxylase (PheH), wobei die Variante eine erhöhte Hydroxylierungsaktivität von mindestens einer aus der TPH, TH and PheH im Vergleich zur nativen *E.-coli*-GCH1 bereitstellt,
wobei
(i) die Variante mindestens 94 % Sequenzidentität mit nativer *E.-coli*-GCH1 mit der Sequenz von SEQ ID NO:16 aufweist und mindestens eine der einen oder mehreren Mutationen in einem Aminosäurerest ist, der ausgewählt ist aus der Gruppe bestehend aus D97, M99, T101, V102, A125, K129, N170, V179, T196, S199, L200, S207, H212, E213, F214, L215 und H221,
wobei die Mutation in N170 N170K, N170D oder N170L ist; die Mutation in V179 V179A ist; die Mutation in H212 H212R oder H212K ist; und die Mutation in H221 H221R oder H221K ist; oder
(ii) die Variante mindestens 98 % Sequenzidentität mit nativer *E.-coli*-GCH1 mit der Sequenz von SEQ ID NO:16 aufweist und mindestens eine der einen oder mehreren Mutationen in dem Aminosäurerest T198 ist, wobei die Mutation in T198 nicht T198P ist.

2. Variante nach Anspruch 1, wobei die Variante
(a) eine Mutation umfasst, ausgewählt aus D97V, D97L, D97A, D97T, M99C, M99T, M99V, M99L, M99I, T101I, T101V, T101L, V102M, N170K, N170D, N170L, V179A, V179M, T196I, T196V, T196L, S199Y, S199F, L200P, L200C, L200S, L200A, S207R, S207K, S207M, H212R, H212K, E213K, E213R, F214A, F214G, F214S, L215P, L215Q, L215N, L215D, L215T, L215S, L215G, L215A, L215C, L215F, L215M, H221R und H221K oder eine Kombination davon;
(b) mindestens 98 % oder mindestens 99 % Sequenzidentität mit der nativen *E*.-coli-GCH1 aufweist;
(c) eine Hydroxylierungsaktivität von mindestens etwa 120 % im Vergleicht zur nativen *E.-coli*-GCH1 bereitstellt; oder
(d) eine Kombination aus beliebigen zwei oder mehreren der Punkte (a) bis (c).

3. Variante nach Anspruch 1, umfassend Aminosäuresubstitutionen, ausgewählt aus
(a) I67V, T117I, A125D, H221R;
(b) V179A;
(c) M99I;
(d) E62K, N170K, L215P;
(e) V102M, L215P;
(f) Q157L, H212R;
(g) V28L, L215P, N222I;
(h) F214S;
(i) T108N, I133F, E213K;
(j) S5C, D57V, L215Q;
(k) H29Y, I75V, V179M;
(l) A14V, E46D, M61I, D97V;
(m)V28A, G42D, E213K;
(n) N52K, A68S, S207R;
(o) L200P;
(p) A41G, K129N, I133F;
(q) S3L, K184R, S199Y; und
(r) H12R, N170D, G187S.

4. Variante nach einem der vorhergehenden Ansprüche, umfassend eine Mutation, ausgewählt aus F214S, V179A, M99I und L200P.

5. Variant nach Anspruch 1, wobei die Variante
(a) eine Mutation umfasst, ausgewählt aus T198I, T198V, T198S und T198L;
(b) mindestens 99 % Sequenzidentität mit der nativen *E.-coli*-GCH1 aufweist;
(c) eine Hydroxylierungsaktivität von mindestens etwa 120 % im Vergleicht zur nativen *E*.-coli-GCH1 bereitstellt; oder
(d) eine Kombination aus beliebigen zwei oder mehreren der Punkte (a) bis (c).

6. Variante einer Tryptophan-Hydroxylase (TPH), umfassend
ein Segment, das den Resten E147 bis T460 von *Homo-sapiens*-TPH mit der Sequenz von SEQ ID NO:3 entspricht,
einen N-terminalen Methioninrest, und
eine Mutation in jedem Rest, der den Resten E147, N242 und P244 in SEQ ID NO:3 entspricht,
wobei die Variante eine Variante einer *Homo-sapiens-* (SEQ ID NO:3), *Schistosoma-mansoni-* (SEQ ID NO:9), *Gallus-gallus-* (SEQ ID NO:6), *Sus- scrofa-* (SEQ ID NO:5), *Equus-caballus-* (SEQ ID NO:8), *Bos-taurus-* (SEQ ID NO:4), *Mus-musculus-* (SEQ ID NO:7) oder *Oryctolagus-cuniculus-* (SEQ ID NO:1):TPH ist, und wobei das Segment in der Variante mindestens etwa 80 % Sequenzidentität mit dem Segment in der nativen Sequenz aufweist,
wobei die Variante eine höhere Tryptophan-Hydroxylierungsaktivität als die native TPH bereitstellt, und
wobei
(a) die Mutation in dem Rest, der dem Rest E147 entspricht, eine Aminosäuresubstitution ist, ausgewählt aus 147K, 147R und 147H;
(b) die Mutation in dem Rest, der dem Rest N242 entspricht, die Aminosäuresubstitution 242I ist; und
(c) die Mutation in dem Rest, der P244 entspricht, ausgewählt ist aus 244C, 244D, 244L und 244Q.

7. Variante nach Anspruch 6, umfassend oder bestehend aus der Aminosäuresequenz von SEQ ID NO: 13 mit den Mutationen E2K, N97I und P99C.

8. Nukleinsäuresequenz, kodierend
(a) die Variante von *E.-coli*-GCH1 nach einem der Ansprüche 1 bis 5;
(b) die *Homo-sapiens*-TPH-Variante nach einem der Ansprüche 6 bis 7; oder
(c) sowohl (a) als auch (b).

9. Rekombinante mikrobielle Zelle, umfassend die *E.-coli*-GCH1-Variante nach einem der Ansprüche 1 bis 5 oder die Nukleinsäuresequenz nach Anspruch 8(a).

10. Rekombinante mikrobielle Zelle nach Anspruch 9, ferner umfassend Nukleinsäuresequenzen, die eine PCD und eine Monooxygenase kodieren, wobei jede Nukleinsäuresequenz funktionell mit einem induzierbaren, einem regulierten oder einem konstitutiven Promoter verbunden ist, und/oder wobei mindestens die Nukleinsäuresequenz, die die Variante von *E.-coli*-GCH1 kodiert, chromosomal integriert ist.

11. Rekombinante mikrobielle Zelle nach Anspruch 10, wobei
(a) die PCD ausgewählt ist aus *Chromobacterium violecum, Homo sapiens, Pseudomonas aeruginosa* und *Rattus norvegicus;* oder einer funktionell aktiven Variante, einem Homolog oder Fragment davon;
(b) die Monooxygenase eine TPH ist, ausgewählt aus einer *Schistosoma-mansoni-, Homo-sapiens-, Gallus-gallus-, Bos-taurus-, Sus-scrofa-, Equus-caballus-, Mus-musculus-* und *Oryctolagus-cuniculus*-TPH; oder einer funktionell aktiven Variante, einem Homolog oder Fragment von einem beliebigen davon, gegebenenfalls die TPH-Variante nach einem der Ansprüche 6 und 7;
(c) die Monooxygenase eine TH ist, ausgewählt aus *Rattus norwegicus, Homo sapiens, Mus musculus, Bos taurus, Gallus gallus* oder einer funktionell aktiven Variante, einem Homolog oder Fragment davon;
(d) die Monooxygenase eine PheH ist, ausgewählt aus *Chromobacterium violaceum, Xanthomonas campestris pv. Viticola, Pseudomonas aeruginosa, Pseudomonas putida, Homo sapiens, Mus musculus, Streptomyces coeruleorubidus* oder einer funktionell aktiven Variante, einem Homolog oder Fragment davon; oder
(e) einer Kombination von (a) und (b), (a) und (c) oder (a) und (d).

12. Rekombinante mikrobielle Zelle nach einem der Ansprüche 9 bis 11, die von einer Bakterienzelle, einer Hefezelle, einer filamentösen Pilzzelle oder einer Algenzelle abgeleitet ist.

13. Rekombinante mikrobielle Zelle nach einem der Ansprüche 9 bis 12, die von einer *Escherichia-,* einer *Saccharomyces-,* einer *Schizosaccharomyces-,* einer *Corynebacterium-,* einer *Bacillus-* oder einer *Streptomyces-Zelle,* wie etwa einer *E*.-coli-Zelle, abgeleitet ist.

14. Rekombinante mikrobielle Zelle nach einem der Ansprüche 9 bis 13, ferner umfassend
(a) eine Nukleinsäuresequenz, die eine 5HTP-Decarboxylase (ADDC) kodiert;
(b) Nukleinsäuresequenzen, die eine ADDC und eine Serotonin-Acetyltransferase (AANAT) kodieren;
(c) Nukleinsäuresequenzen, die eine ADDC, eine AANAT und eine Acetylserotonin-O-methyltransferase (ASMT) kodieren; oder
(d) Nukleinsäuresequenzen, die eine Dopa-Decarboxylase, eine Tyraminoxidase und eine Alkoholdehydrogenase kodieren.

15. Verfahren zur Herstellung eines oder mehrerer Oxidationsprodukte einer aromatischen Aminosäure, umfassend das Kultivieren der rekombinanten mikrobiellen Zelle nach einem der Ansprüche 9 bis 14 in einem Medium, umfassend eine Kohlenstoffquelle, und gegebenenfalls das Isolieren des Oxidationsprodukts.

16. Verfahren nach Anspruch 15, wobei das Oxidationsprodukt mindestens eines von 5HTP, L-DOPA, Tyrosin, m-Tyrosin, Hydroxytyrosol, Serotonin und Melatonin umfasst.

## Revendications

1. Variant de la GTP cyclohydrolase I *d'E. coli* (GCH1) comprenant une ou plusieurs mutations, dans lequel,
dans une cellule *d'E. coli* comprenant une ptérine-4a-carbinolamine déshydratase (PCD) et au moins l'une d'une tryptophane hydroxylase (TPH), d'une tyrosine hydroxylase (TH) et d'une phénylalanine hydroxylase (PheH), le variant fournit une activité d'hydroxylation accrue d'au moins l'une de la TPH, de la TH et de la PheH comparé à une GCH1 *d'E. coli* native,
dans lequel
(i) le variant présente une identité de séquence d'au moins 94 % avec la GCH1 *d'E. coli* native ayant la séquence de SEQ ID NO : 16 et au moins l'une des une ou plusieurs mutations est dans un résidu d'acide aminé sélectionné parmi le groupe constitué de D97, M99, T101, V102, A125, K129, N170, V179, T196, S199, L200, S207, H212, E213, F214, L215 et H221,
dans lequel la mutation dans N170 est N170K, N170D ou N170L ; la mutation dans V179 est V179A ; la mutation dans H212 est H212R ou H212K ; et la mutation dans H221 est H221R ou H221K ; ou
(ii) le variant présente une identité de séquence d'au moins 98 % avec la GCH1 *d'E. coli* native ayant la séquence de SEQ ID NO : 16 et au moins l'une des une ou plusieurs mutations est dans le résidu d'acide aminé T198, dans lequel la mutation dans T198 n'est pas T198P.

2. Variant selon la revendication 1, dans lequel le variant
(a) comprend une mutation sélectionnée parmi D97V, D97L, D97A, D97T, M99C, M99T, M99V, M99L, M99I, T101I, T101V, T101L, V102M, N170K, N170D, N170L, V179A, V179M, T196I, T196V, T196L, S199Y, S199F, L200P, L200C, L200S, L200A, S207R, S207K, S207M, H212R, H212K, E213K, E213R, F214A, F214G, F214S, L215P, L215Q, L215N, L215D, L215T, L215S, L215G, L215A, L215C, L215F, L215M, H221R et H221K, ou une combinaison de celles-ci ;
(b) présente une identité de séquence d'au moins 98 % ou d'au moins 99 % avec la GCH1 *d'E. coli* native ;
(c) fournit une activité d'hydroxylation d'au moins environ 120 % comparé à la GCH1 *d'E. coli* native ; ou
(d) une combinaison de deux quelconques ou plus de (a) à (c).

3. Variant selon la revendication 1, comprenant des substitutions d'acides aminés sélectionnées parmi
(a) I67V, T117I, A125D, H221R ;
(b) V179A ;
(c) M99I ;
(d) E62K, N170K, L215P ;
(e) V102M, L215P ;
(f) Q157L, H212R ;
(g) V28L, L215P, N222I ;
(h) F214S ;
(i) T108N, I133F, E213K ;
(j) S5C, D57V, L215Q ;
(k) H29Y, I75V, V179M ;
(l) A14V, E46D, M61I, D97V ;
(m)V28A, G42D, E213K ;
(n) N52K, A68S, S207R ;
(o) L200P ;
(p) A41G, K129N, I133F ;
(q) S3L, K184R, S199Y ; et
(r) H12R, N170D, G187S.

4. Variant selon l'une quelconque des revendications précédentes, comprenant une mutation sélectionnée parmi F214S, V179A, M99I et L200P.

5. Variant selon la revendication 1, dans lequel le variant
(a) comprend une mutation sélectionnée parmi T198I, T198V, T198S et T198L ;
(b) présente une identité de séquence d'au moins 99 % avec la GCH1 *d'E. coli* native ;
(c) fournit une activité d'hydroxylation d'au moins environ 120 % comparé à la GCH1 *d'E. coli* native ; ou
(d) une combinaison de deux quelconques ou plus de (a) à (c).

6. Variant d'une tryptophane hydroxylase (TPH), comprenant
un segment correspondant aux résidus E147 à T460 de la TPH d'*Homo sapiens* ayant la séquence de SEQ ID NO : 3,
un résidu méthionine N-terminal, et
une mutation dans chaque résidu correspondant aux résidus E147, N242 et P244 dans SEQ ID NO : 3,
dans lequel le variant est un variant d'une TPH d'*Homo sapiens* (SEQ ID NO : 3), de *Schistosoma mansoni* (SEQ ID NO : 9), de *Gallus gallus* (SEQ ID NO : 6), de *Sus scrofa* (SEQ ID NO : 5), *d'Equus caballus* (SEQ ID NO : 8), de *Bos taurus* (SEQ ID NO : 4), de *Mus musculus* (SEQ ID NO : 7) ou *d'Oryctolagus cuniculus* (SEQ ID NO : 1), et dans lequel le segment dans le variant présente une identité de séquence d'au moins environ 80 % avec le segment dans la séquence native,
dans lequel le variant fournit une activité d'hydroxylation du tryptophane plus élevée que la TPH native, et
dans lequel
(a) la mutation dans le résidu correspondant au résidu E147 est une substitution d'acide aminé sélectionnée parmi 147K, 147R et 147H ;
(b) la mutation dans le résidu correspondant au résidu N242 est la substitution d'acide aminé 242I ; et
(c) la mutation dans le résidu correspondant à P244 est sélectionnée parmi 244C, 244D, 244L et 244Q.

7. Variant selon la revendication 6, comprenant ou consistant en la séquence d'acides aminés de SEQ ID NO : 13 avec les mutations E2K, N97I et P99C.

8. Séquence d'acide nucléique codant pour
(a) le variant de la GCH1 *d'E. coli* selon l'une quelconque des revendications 1 à 5 ;
(b) le variant de la TPH d'*Homo sapiens* selon l'une quelconque des revendications 6 à 7 ; ou
(c) à la fois (a) et (b).

9. Cellule microbienne recombinante comprenant le variant de la GCH1 *d'E. coli* selon l'une quelconque des revendications 1 à 5 ou la séquence d'acide nucléique selon la revendication 8(a).

10. Cellule microbienne recombinante selon la revendication 9, comprenant en outre des séquences d'acide nucléique codant pour une PCD et une monooxygénase, facultativement dans laquelle chaque séquence d'acide nucléique est fonctionnellement liée à un promoteur inductible, régulé ou constitutif, et/ou dans laquelle au moins la séquence d'acide nucléique codant pour le variant de la GCH1 *d'E. coli* est chromosomiquement intégrée.

11. Cellule microbienne recombinante selon la revendication 10, dans laquelle
(a) la PCD est sélectionnée parmi *Chromobacterium violecum, Homo sapiens, Pseudomonas aeruginosa* et *Rattus norvegicus* ; ou un variant, un homologue ou un fragment fonctionnellement actifs de celles-ci ;
(b) la monooxygénase est une TPH sélectionnée parmi une TPH de *Schistosoma mansoni,* d'*Homo sapiens,* de *Gallus gallus,* de *Bos taurus,* de *Sus scrofa, d'Equus caballus,* de *Mus musculus* et *d'Oryctolagus cuniculus* ; ou un variant, un homologue ou un fragment fonctionnellement actifs de l'une quelconque de celles-ci, facultativement le variant de la TPH selon l'une quelconque des revendications 6 et 7 ;
(c) la monooxygénase est une TH sélectionnée parmi *Rattus norwegicus, Homo sapiens, Mus musculus, Bos taurus, Gallus gallus* ou un variant, homologue ou fragment fonctionnellement actifs de celles-ci ;
(d) la monooxygénase est une PheH sélectionnée parmi *Chromobacterium violaceum, Xanthomonas campestris pv. Viticola, Pseudomonas aeruginosa, Pseudomonas putida, Homo sapiens, Mus musculus, Streptomyces coeruleorubidus* ou un variant, homologue ou fragment fonctionnellement actifs de celles-ci ; ou
(e) une combination de (a) et (b), (a) et (c) ou (a) et (d).

12. Cellule microbienne recombinante selon l'une quelconque des revendications 9 à 11, qui est dérivée d'une cellule bactérienne, d'une cellule de levure, d'une cellule fongique filamenteuse, ou d'une cellule d'algue.

13. Cellule microbienne recombinante selon l'une quelconque des revendications 9 à 12, qui est dérivée d'une cellule d*'Escherichia,* de *Saccharomyces,* de *Schizosaccharomyces,* de *Corynebacterium,* de *Bacillus* ou de *Streptomyces,* par exemple une cellule *d'E. coli.*

14. Cellule microbienne recombinante selon l'une quelconque des revendications 9 à 13, comprenant en outre
(a) une séquence d'acide nucléique codant pour une 5HTP décarboxylase (ADDC) ;
(b) des séquences d'acide nucléique codant pour une ADDC et une sérotonine acétyltransférase (AANAT) ;
(c) des séquences d'acide nucléique codant pour une ADDC, une AANAT, et une acétylsérotonine O-méthyltransférase (ASMT) ; ou
(d) des séquences d'acide nucléique codant pour une dopa décarboxylase, une tyramine oxydase et une alcool déshydrogénase.

15. Procédé de production d'un ou plusieurs produits d'oxydation d'un acide aminé aromatique, comprenant la culture de la cellule microbienne recombinante selon l'une quelconque des revendications 9 à 14 dans un milieu comprenant une source de carbone, et, facultativement, l'isolation du produit d'oxydation.

16. Procédé selon la revendication 15, dans lequel le produit d'oxydation comprend au moins l'un de la 5HTP, de la L-DOPA, de la tyrosine, de la m-tyrosine, de l'hydroxytyrosol, de la sérotonine et de la mélatonine.
